(19) Europäisches Patentamt
European Patent Office
Office européen des brevets

(11) EP 3 142 771 B1

(12) **FASCICULE DE BREVET EUROPEEN**

(45) Date de publication et mention de la délivrance du brevet:
**12.09.2018 Bulletin 2018/37**

(21) Numéro de dépôt: **15721724.1**

(22) Date de dépôt: **12.05.2015**

(51) Int Cl.:
***B01D 53/14*** (2006.01) ***B01D 53/52*** (2006.01)
***C07C 213/04*** (2006.01) ***C07C 215/18*** (2006.01)

(86) Numéro de dépôt international:
**PCT/EP2015/060516**

(87) Numéro de publication internationale:
**WO 2015/173262 (19.11.2015 Gazette 2015/46)**

(54) **DIAMINES TERTIAIRES BETA-HYDROXYLEES, LEUR PROCEDE DE SYNTHESE ET LEUR UTILISATION POUR L'ÉLIMINATION DE COMPOSES ACIDES D'UN EFFLUENT GAZEUX**

BETA-HYDROXYLIERTE TERTIÄRE DIAMINE, VERFAHREN ZU DEREN SYNTHESE UND DEREN VERWENDUNG ZUR BESEITIGUNG VON SÄUREVERBINDUNGEN AUS EINEM GASFÖRMIGEM ABSTROM

BETA-HYDROXYLATED TERTIARY DIAMINES, A PROCESS FOR THEIR SYNTHESIS AND THEIR USE FOR ELIMINATING ACID COMPOUNDS FROM A GASEOUS EFFLUENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.05.2014 FR 1454371**

(43) Date de publication de la demande:
**22.03.2017 Bulletin 2017/12**

(73) Titulaire: **IFP Energies nouvelles**
**92500 Rueil-Malmaison (FR)**

(72) Inventeurs:
- **DELFORT, Bruno**
  **F-75005 Paris (FR)**
- **LE PENNEC, Dominique**
  **F-78910 Orgerus (FR)**
- **GRANDJEAN, Julien**
  **F-69008 Lyon (FR)**
- **HUARD, Thierry**
  **F-69360 Saint Symphorien d'Ozon (FR)**
- **WENDER, Aurélie**
  **F-92500 Rueil-Malmaison (FR)**
- **NIGON, Armelle**
  **F-92500 Rueil-Malmaison (FR)**

(56) Documents cités:
**WO-A1-2013/060944**
**DE-A1- 2 320 189**
**US-A- 3 867 454**
**US-A- 5 277 885**
**US-A1- 2010 105 551**
**US-A1- 2013 243 677**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 142 771 B1

## Description

### Domaine de l'invention

La présente invention concerne de nouveaux composés azotés appartenant à la famille des diamines tertiaires béta-hydroxylées. L'invention concerne également le procédé de synthèse desdits composés, ainsi que leur utilisation dans un procédé de désacidification d'un effluent gazeux.

### Contexte général

Les amines tertiaires, notamment certaines diamines tertiaires, présentent un intérêt pour différentes applications. Elles peuvent par exemple être utilisées comme catalyseurs dans la préparation de polyuréthanes, comme précurseurs de sels d'ammonium quaternaires ou comme bases lors de la désacidification de gaz acides.

Les amines tertiaires peuvent être utilisées dans des procédés de désacidification de gaz acides mettant en oeuvre une solution aqueuse comprenant de telles aminés, pour retirer les composés acides, notamment le dioxyde de carbone ($CO_2$), l'hydrogène sulfuré ($H_2S$), l'oxysulfure de carbone (COS), le disulfure de carbone ($CS_2$), le dioxyde de soufre ($SO_2$) et les mercaptans (RSH) tel que le méthylmercaptan ($CH_3SH$), l'éthylmercaptan ($CH_3CH_2SH$) et le propylmercaptan ($CH_3CH_2CH_2SH$), présents dans un gaz. Le gaz est désacidifié par mise en contact avec la solution absorbante, puis la solution absorbante est régénérée thermiquement.

Ces procédés de désacidification de gaz acides sont aussi communément appelés "lavage aux solvants" avec un solvant dit "chimique", par opposition à l'utilisation solvant dit "physique" pour l'absorption qui n'est pas basée sur la réalisation de réactions chimiques.

Un solvant chimique correspond à une solution aqueuse comprenant un réactif qui réagit sélectivement avec les composés acides ($H_2S$, $CO_2$, COS, $CS_2$, etc.) présents dans le gaz traité pour former des sels, sans réagir avec les autres composés non acides du gaz. Le gaz traité après mise en contact avec le solvant est alors appauvri en composés acides, lesquels sont sélectivement transférés sous forme de sels dans le solvant. Les réactions chimiques sont réversibles, ce qui permet au solvant chargé en composés acides d'être ensuite désacidifié, par exemple sous l'action de la chaleur, pour libérer d'une part les composés acides sous forme de gaz, qui peuvent alors être stockés, transformés ou être utilisés pour diverses applications, et pour d'autre part régénérer le solvant qui retourne à son état initial et peut ainsi être réutilisé pour une nouvelle phase de réaction avec le gaz acide à traiter. La phase de réaction du solvant avec le gaz acide est communément appelée la phase d'absorption, et celle où le solvant est désacidifié est appelée la phase de régénération du solvant.

En général, les performances de la séparation des composés acides du gaz, dans ce contexte, dépendent principalement de la nature de la réaction réversible choisie. Les procédés conventionnels de désacidification de gaz acides sont généralement des procédés dits "aux aminés", c'est à dire qu'ils reposent sur les réactions des composés acides avec des amines en solution. Ces réactions entrent dans le cadre général des réactions acido-basiques. L'$H_2S$, le $CO_2$, ou le COS sont par exemple des composés acides, notamment en présence d'eau, alors que les amines sont des composés basiques. Les mécanismes des réactions et la nature des sels obtenus dépendent généralement de la structure des amines mises en oeuvre.

Par exemple le document US 6 852 144 décrit une méthode d'élimination des composés acides des hydrocarbures utilisant une solution absorbante eau-N-méthyldiéthanolamine ou eau-triéthanolamine contenant une forte proportion d'un composé appartenant au groupe suivant pipérazine et/ou méthylpipérazine et/ou morpholine.

Les performances des procédés de désacidification de gaz acides par lavage aux aminés sont directement dépendantes de la nature de la ou des amines présentes dans le solvant. Ces amines peuvent être primaires, secondaires ou tertiaires. Elles peuvent présenter une ou plusieurs fonctions amines équivalentes ou différentes par molécule.

Afin d'améliorer les performances des procédés de désacidification, il est continuellement recherché des amines toujours plus performantes.

Une limitation des solutions absorbantes couramment utilisées dans des applications de désacidification est une sélectivité insuffisante d'absorption de l'$H_2S$ par rapport au $CO_2$. En effet, dans certains cas de désacidification du gaz naturel, on recherche une élimination sélective de l'$H_2S$ en limitant au maximum l'absorption du $CO_2$. Cette contrainte est particulièrement importante pour des gaz à traiter contenant déjà une teneur en $CO_2$ inférieure ou égale à la spécification désirée. On recherche alors une capacité d'absorption de $H_2S$ maximale avec une sélectivité maximale d'absorption de $H_2S$ vis-à-vis du $CO_2$. Cette sélectivité permet de maximiser la quantité de gaz traité et de récupérer un gaz acide en sortie de régénérateur ayant une concentration la plus élevée possible en $H_2S$, ce qui limite la taille des unités de la chaîne soufre en aval du traitement et garantit un meilleur fonctionnement. Dans certains cas, une unité d'enrichissement en $H_2S$ est nécessaire pour concentrer en $H_2S$ le gaz acide. Dans ce cas, on recherche également l'amine la plus sélective. Des amines tertiaires, comme la N-méthyldiéthanolamine, ou des amines secondaires encombrées présentant une cinétique de réaction lente avec le $CO_2$ sont couramment utilisées, mais présentent des sélectivités

limitées à des taux de charge en $H_2S$ élevés.

Il est bien connu de l'homme du métier que les amines tertiaires ou les amines secondaires avec un encombrement stérique sévère ont une cinétique de captage du $CO_2$ plus lente que des amines primaires ou secondaires peu encombrées. En revanche, les amines tertiaires ou secondaires avec un encombrement stérique sévère ont une cinétique de captage de l'$H_2S$ instantanée, ce qui permet de réaliser une élimination sélective de l'$H_2S$ basée sur des performances cinétiques distinctes.

Différents documents proposent d'utiliser des amines tertiaires ou secondaires encombrées, en particulier des diamines tertiaires, en solution pour désacidifier des gaz acides.

Parmi les applications des amines tertiaires ou secondaires avec un encombrement stérique sévère, le brevet US 4,405,582 décrit un procédé d'absorption sélective de gaz sulfurés par un absorbant contenant un diaminoéther dont au moins une fonction amine est tertiaire et dont l'autre fonction amine est tertiaire ou secondaire possédant un encombrement stérique sévère, l'atome d'azote étant dans ce dernier cas lié soit à au moins un carbone tertiaire, soit à deux atomes de carbone secondaires. Les deux fonctions amines et les carbones de la chaîne principale peuvent être substitués par des radicaux alkyles ou hydroxyalkyles.

Le brevet US 4,405,583 décrit également un procédé d'élimination sélective de l'$H_2S$ dans des gaz contenant de l'$H_2S$ et du $CO_2$ par un absorbant contenant un diaminoéther dont les deux fonctions amines secondaires présentent un encombrement stérique sévère défini comme précédemment. Les substituants des fonctions amines et des carbones de la chaîne principale peuvent être substitués par des radicaux alkyle et hydroxyalkyle.

Le brevet FR 2934172 décrit la mise en oeuvre d'une solution absorbante à base d'une diamine tertiaire dans un procédé d'élimination de composés acides s'appliquant avantageusement au traitement du gaz naturel et aux fumées de combustion, ladite amine étant la N,N,N',N'-tétraméthyl-1,6-hexanediamine.

WO2013/060944, US2013/243677, US2010/105551 et US5277885 décrivent aussi l'utilisation de différents composés aminoalcools ou aminoethers pour désacidifier des effluents gazeux comprenant $H_2S$ et/ou $CO_2$.

Une autre limitation des solutions absorbantes couramment utilisées dans des applications de désacidification totale est une cinétique de captage du $CO_2$ ou du COS trop lente. Dans le cas où les spécifications désirées sur le $CO_2$ ou en COS sont très poussées, on recherche une cinétique de réaction la plus rapide possible de manière à réduire la hauteur de la colonne d'absorption. Cet équipement sous pression représente en effet une part importante des coûts d'investissements du procédé.

Que l'on recherche une cinétique de captage du $CO_2$ et du COS maximale dans une application désacidification totale, ou une cinétique de captage du $CO_2$ minimale dans une application sélective, on désire toujours utiliser une solution absorbante ayant la capacité cyclique la plus grande possible. Cette capacité cyclique, notée $\Delta\alpha$ correspond à la différence de taux de charge ($\alpha$ désignant le nombre de mole de composés acides absorbés $n_{gaz\ acide}$ par kilogramme de solution absorbante) entre la solution absorbante soutirée en fond de la colonne d'absorption et la solution absorbante alimentant ladite colonne. En effet, plus la solution absorbante a une forte capacité cyclique, plus le débit de solution absorbante qu'il faut mettre en oeuvre pour désacidifier de gaz à traiter est restreint. Dans les procédés de traitement de gaz, la réduction du débit de solution absorbante a également un fort impact sur la réduction des investissements, notamment au niveau du dimensionnement de la colonne d'absorption.

Un autre aspect primordial des opérations de traitement de gaz ou fumées industrielles par solvant reste la régénération de l'agent de séparation. En fonction du type d'absorption (physique et/ou chimique), on envisage généralement une régénération par détente, et/ou par distillation et/ou par entraînement par un gaz vaporisé appelé "gaz de strippage". La consommation énergétique nécessaire à la régénération du solvant peut être très importante, ce qui est particulièrement vrai dans le cas où la pression partielle de gaz acides est faible, et représenter un coût opératoire considérable pour le procédé de captage du $CO_2$.

Il est bien connu de l'homme du métier que l'énergie nécessaire à la régénération par distillation d'une solution d'amine peut se décomposer selon trois postes différents : l'énergie nécessaire pour réchauffer la solution absorbante entre la tête et le fond du régénérateur, l'énergie nécessaire pour abaisser la pression partielle de gaz acide dans le régénérateur par vaporisation d'un gaz de strippage, et enfin l'enthalpie nécessaire pour casser la liaison chimique entre l'amine et le $CO_2$.

Ces deux premiers postes sont proportionnels aux débits de solution absorbante qu'il est nécessaire de faire circuler dans l'unité pour réaliser une spécification donnée. Pour diminuer la consommation énergétique associée à la régénération du solvant, il est donc préférable encore une fois de maximiser la capacité cyclique du solvant. En effet, plus la solution absorbante a une forte capacité cyclique, plus le débit de solution absorbante qu'il faut mettre en oeuvre pour désacidifier de gaz à traiter est restreint.

Il existe ainsi un besoin, dans le domaine de la désacidification de gaz, de fournir des composés qui soient de bons candidats pour l'élimination des composés acides d'un effluent gazeux, notamment, mais pas exclusivement, pour l'élimination sélective de l'$H_2S$ par rapport au $CO_2$, et qui permettent de fonctionner à moindres coûts opératoires (dont l'énergie de régénération) et d'investissements (dont le coût de la colonne d'absorption).

## Description de l'invention

Les inventeurs ont découvert de nouveaux composés azotés appartenant à la famille des diamines tertiaires béta-hydroxylées, pouvant être avantageusement utilisés dans le domaine de la désacidification de gaz.

Les inventeurs ont mis en évidence que les diamines tertiaires ou secondaires possédant un encombrement stérique sévère ne sont pas équivalentes en terme de performances pour leur usage dans des formulations de solutions absorbantes pour le traitement de gaz acides dans un procédé industriel.

Les nouveaux composés azotés selon l'invention sont des diamines tertiaires particulières, dont la chaîne principale, c'est-à-dire la chaîne reliant les deux fonctions amines tertiaires, est une chaine hydrocarbonée substituée par deux groupements hydroxyles, chacun des groupements hydroxyle étant porté par un atome de carbone situé en position beta des atomes d'azote. On utilisera la désignation de diamines tertiaires béta-hydroxylées dans la présente invention en référence à la position d'un groupement hydroxyle par rapport à une fonction amine tel que décrit.

## Résumé de l'invention

L'invention porte, selon un premier aspect, sur un composé azoté appartenant à la famille des diamines tertiaires répondant à la formule générale (I) suivante :

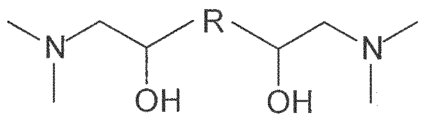

(I)

dans laquelle R est un radical alcanediyl $-(CH_2)_n-$ avec n = 2, 3, 4, 5 ou 6.

De préférence, le composé azoté selon l'invention est du N,N,N',N'-(tétraméthyl)-1,6-diamino-2,5-hexanediol et répond à la formule suivante, avec n étant égal à 2 et R étant un radical éthylidène:

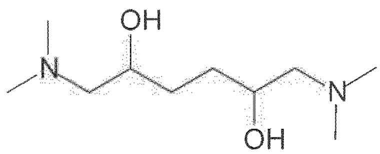

De préférence, le composé azoté selon l'invention est du N,N,N',N'-(tétraméthyl)-1,8-diamino-2,7-octanediol et répond à la formule suivant, avec n étant égal à 4 et R étant un radical butylidène :

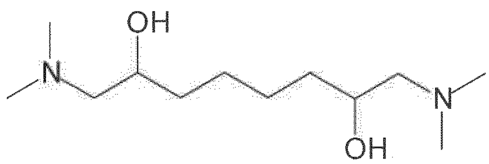

L'invention porte, selon un deuxième aspect, sur un procédé de synthèse d'un composé azoté selon la formule générale (I), comprenant les réactions suivantes :

- une première réaction d'époxydation d'un alpha-oméga-diène pour effectuer l'époxydation de chacune des fonctions alcènes de l'alpha-oméga-diène en fonctions oxiranes afin de produire un diépoxyalcane;
- une deuxième réaction d'addition de deux moles de diméthylamine et d'une molécule du diépoxyalcane pour produire le composé azoté selon la formule générale (I).

Selon un mode de réalisation, la première réaction est une réaction d'époxydation du 1,5-hexadiène pour produire du 1,2,5,6-diépoxyhexane, et la deuxième réaction est une réaction d'addition de deux moles de diméthylamine et d'une molécule de 1,2,5,6-diépoxyhexane pour produire du N,N,N',N'-(tétraméthyl)-1,6-diamino-2,5-hexanediol.

Selon un mode de réalisation, la première réaction est une réaction d'époxydation du 1,7-octadiène pour produire du 1,2,7,8-diépoxyoctane, et la deuxième réaction est une réaction d'addition de deux moles de diméthylamine et d'une molécule de 1,2,7,8-diépoxyoctane pour produire du N,N,N',N'-(tétraméthyl)-1,8-diamino-2,7-octanediol.

De préférence, la première réaction d'époxydation consiste à faire réagir l'alpha-oméga-diène avec un peracide, ou un peroxyde, ou un hydroperoxyde ou de l'oxygène associé à un système catalytique approprié, ledit peracide pouvant être généré in situ par réaction entre un acide carboxylique et un peroxyde d'hydrogène.

Avantageusement, la deuxième réaction d'addition est opérée en présence d'un excès de diméthylamine, de préférence plus de deux moles.

Selon un mode de réalisation, la première réaction d'époxydation et la deuxième réaction d'addition sont conduites en deux étapes successives.

L'invention porte, selon un troisième aspect, sur un procédé d'élimination des composés acides contenus dans un effluent gazeux dans lequel on effectue une étape d'absorption des composés acides par mise en contact de l'effluent gazeux avec une solution absorbante comportant de l'eau et un composé azoté selon l'invention ou susceptible d'être obtenu par un procédé de synthèse selon l'invention.

De préférence, la solution absorbante comporte entre 5 % et 95 % poids dudit composé azoté, de préférence entre 10 % et 90 % poids dudit composé azoté, et entre 5 % et 95 % poids d'eau, et de préférence entre 10 % et 90% poids d'eau.

La solution absorbante peut comporter en outre entre 5 % et 95 % poids d'au moins une amine supplémentaire, ladite amine supplémentaire étant soit une amine tertiaire, soit une amine secondaire comprenant deux carbones secondaires en alpha de l'atome d'azote ou au moins un carbone tertiaire en alpha de l'atome d'azote.

Ladite amine supplémentaire peut être une amine tertiaire choisie dans le groupe constitué par :

- la N-méthyldiéthanolamine ;
- la triéthanolamine ;
- la diéthylmonoéthanolamine ;
- la diméthylmonoéthanolamine ; et
- l'éthyldiéthanolamine.

La solution absorbante peut comporter, en outre, une quantité non nulle et inférieure à 30 % poids d'au moins une amine supplémentaire étant une amine primaire ou une amine secondaire.

Ladite amine supplémentaire primaire ou secondaire peut être choisie dans le groupe constitué par :

- la monoéthanolamine ;
- la diéthanolamine ;
- la N-butyléthanolamine ;
- l'aminoéthyléthanolamine ;
- le diglycolamine ;
- la pipérazine ;
- la 1-méthylpipérazine ;
- la 2-méthylpipérazine ;
- l'homopipérazine ;
- la N-(2-hydroxyéthyl)pipérazine ;
- la N-(2-aminoéthyl)pipérazine ;
- la morpholine ;
- la 3-(méthylamino)propylamine ;
- la 1,6-hexanediamine ;
- la N,N,-diméthyl-1,6-hexanediamine ;
- la N,N'-diméthyl-1,6-hexanediamine ;
- la N-méthyl-1,6-hexanediamine ; et
- la N,N',N'-triméthyl-1,6-hexanediamine.

La solution absorbante peut comporter en outre au moins un solvant physique choisi dans le groupe constitué par le méthanol, l'éthanol, le 2-éthoxyéthanol, le triéthylèneglycoldiméthyléther, le tétraéthylèneglycoldiméthyléther, le pentaéthylèneglycoldiméthyléther, l' hexaéthylèneglycoldiméthyléther , l'heptaéthylèneglycoldiméthyléther, l' octaéthylèneglycoldiméthyléther, le butoxyacétate de diéthylèneglycol, le triacétate de glycérol, le sulfolane, la N-méthylpyrrolidone, la N-méthylmorpholin-3-one, le N,N-diméthylformamide, la N-formyl-morpholine, la N,N-diméthyl-imidazolidin-2-one, le N-méthylimidazole, l'éthylèneglycol, le diéthylèneglycol, le triéthylèneglycol, le thiodiglycol, et le tributylphosphate.

L'effluent gazeux peut être choisi parmi le gaz naturel, les gaz de synthèse, les fumées de combustion, les gaz de raffinerie, les gaz acides issus d'une unité aux amines, les gaz issus d'une unité de réduction en queue du procédé Claus, les gaz de fermentation de biomasse, les gaz de cimenterie, les fumées d'incinérateur.

Le procédé selon l'invention peut être mis en oeuvre pour éliminer sélectivement de l'$H_2S$ par rapport au $CO_2$

d'un effluent gazeux comportant de l'$H_2S$ et du $CO_2$, de préférence du gaz naturel.

D'autres objets et avantages de l'invention apparaîtront à la lecture de la description qui suit d'exemples de réalisations particuliers de l'invention, donnés à titre d'exemples non limitatifs, la description étant faite en référence à la figure annexée décrite ci-après.

## Brève description de la figure

La figure 1 représente un schéma de principe de mise en oeuvre d'un procédé de traitement de gaz acides.

Dans les schémas de la présente description illustrant la préparation des composés azotés selon l'invention, les flèches représentent des étapes de réaction. Il s'agit de schémas réactionnels.

## Description détaillée de l'invention

Les nouveaux composés azotés selon l'invention sont des diamines tertiaires répondant à la formule générale (I) suivante :

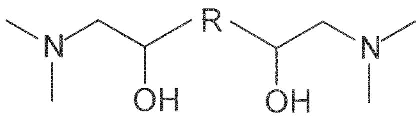

(I)

dans laquelle R est un radical alcanediyl $-(CH_2)n-$ avec n = 2, 3, 4, 5 ou 6.

Par diamine tertiaire, on entend dans la présente description un composé chimique comportant deux fonctions aminés qui sont des fonctions amines tertiaires.

Dans la formule générale (I), les groupements hydroxyles sont portés par des atomes de carbone situés en position beta des fonctions amines.

R est choisi parmi l'un des groupements suivants :

- r1 : un radical 1,2-éthanediyl $-CH_2-CH_2-$ quand n=2. Le composé azoté est alors le N,N,N',N'-(tétraméthyl)-1,6-diamino-2,5-hexanediol de formule suivante :

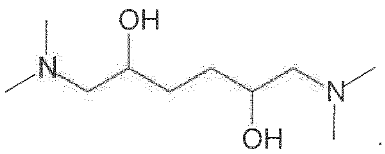

;

- r2 : un radical 1,4-butanediyl $-CH_2-CH_2-CH_2-CH_2-$ quand n=4. Le composé azoté est alors le N,N,N',N'-(tétraméthyl)-1,8-diamino-2,7-octanediol de formule suivante :

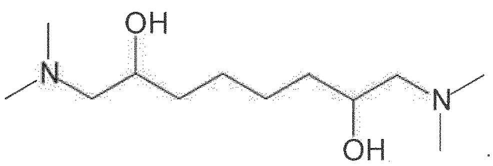

;

- r3 : un radical 1,3-propanediyl quand n=3. Le composé azoté est alors le N,N,N',N'-tétraméthyl-1,7-diamino-2,6-heptanediol de formule suivante :

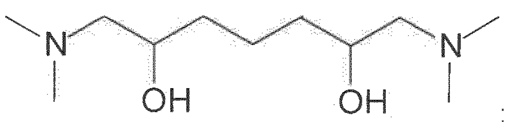

;

- r4: un radical 1,5-pentanediyl quand n=5. Le composé azoté est alors le N,N,N',N'-tétraméthyl-1,9-diamino-2,8-nonanediol de formule suivante :

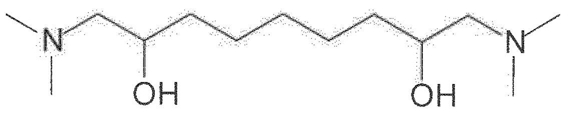

;

- r5 : un radical 1,6-hexanediyl quand n=6. Le composé azoté est alors le N,N,N',N'-tétraméthyl-1,10-diamino-2,9-décanediol de formule suivante :

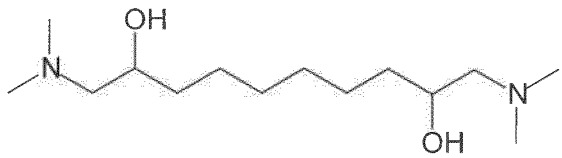

;

Avantageusement, un composé selon l'invention est le N,N,N',N'-(tétraméthyl)-1,6-diamino-2,5-hexanediol ou le N,N,N',N'-(tétraméthyl)-1,8-diamino-2,7-octanediol.

Synthèse d'un composé selon l'invention

Les composés azotés selon la formule générale (I) peuvent être préparés en réalisant les réactions suivantes :

- une première réaction d'époxydation d'un alpha-oméga-diène pour effectuer une époxydation de chacune des fonctions alcènes de l'alpha-oméga-diène en fonctions oxiranes afin de produire un diépoxyalcane ;
- une deuxième réaction d'addition de deux moles de diméthylamine et d'une molécule du diépoxyalcane pour produire le composé azoté selon la formule générale (I).

Par alpha-oméga-diène on entend un diène comportant deux fonctions alcènes aux extrémités, tel que le 1,5 hexadiène et 1,7-octadiène.

La synthèse du N,N,N',N'-(tétraméthyl)-1,6-diamino-2,5-hexanediol est illustrée par le schéma A suivant :

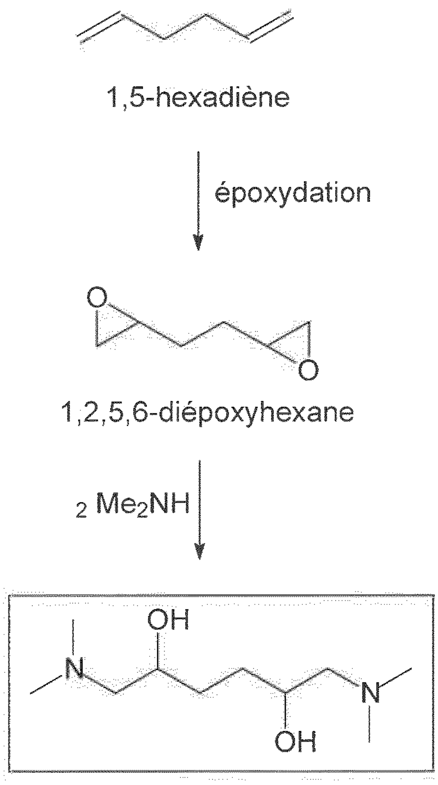


1,5-hexadiène

1,2,5,6-diépoxyhexane

N,N,N',N'-tetraméthyl-1,6-diamino-2,5-hexanediol

*Schéma A : préparation du composé N,N,N',N'-(tétraméthyl)-1,6-diamino-2,5-hexanediol*

Dans un premier temps, on effectue une réaction d'époxydation de chacune des deux fonctions alcènes du 1,5-hexadiène en fonctions oxiranes afin d'obtenir le 1,2,5,6-diépoxyhexane. Cette réaction d'époxydation peut être réalisée avec tous les moyens connus de l'homme du métier pour réaliser l'époxydation d'une double liaison carbone-carbone. On peut par exemple utiliser un peroxyde, un hydroperoxyde, un peracide, tel que l'acide peracétique ou l'acide-3-chloroperbenzoique, ou un perester. On peut également utiliser la combinaison d'un acide tel l'acide acétique et un peroxyde tel le peroxyde d'hydrogène, permettant de générer in situ un peracide. La réaction peut s'effectuer dans des conditions douces, par exemple à une température proche de l'ambiante, et peut se faire en présence d'un solvant, qui peut être un solvant chloré tel que le dichlorométhane, ou un solvant hydrocarboné aliphatique ou aromatique. On peut également réaliser la réaction d'époxydation d'une insaturation au moyen d'oxygène et d'un système catalytique approprié.

Dans un second temps, on réalise une réaction d'addition de deux moles de diméthylamine sur une molécule de 1,2,5,6-diépoxyhexane pour conduire au N,N,N',N'-(tétraméthyl)-1,6-diamino-2,5-hexanediol. Cette réaction peut être conduite avec un excès de diméthylamine. C'est une réaction exothermique qui est réalisée de préférence avec un contrôle de température approprié. Par exemple, la température est maintenue dans la gamme -15°C / 100°C.

La synthèse du N,N,N',N'-(tétraméthyl)-1,8-diamino-2,7-octanediol est illustrée par le schéma B suivant :

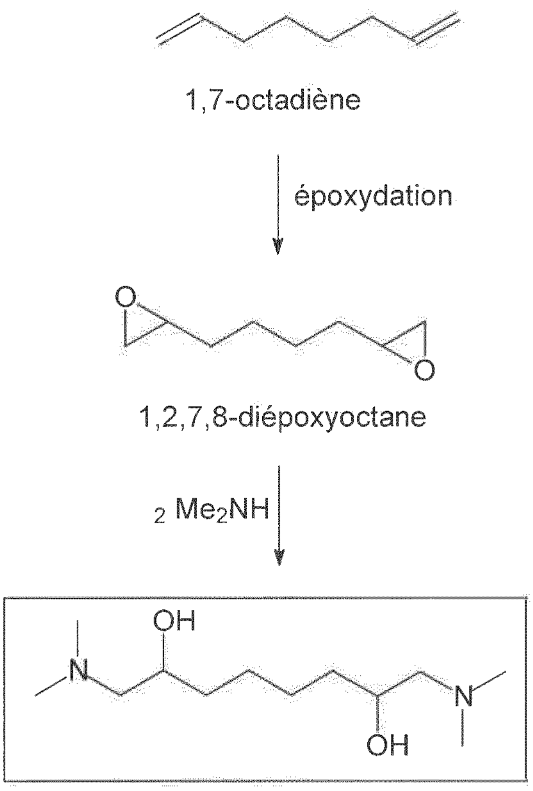


1,7-octadiène

1,2,7,8-diépoxyoctane

N,N,N',N'-tetraméthyl-1,8-diamino-2,7-octanediol

*Schéma B : préparation du composé N,N,N',N'-(tétraméthyl)-1,8-diamino-2,7-octanediol*

Cette synthèse repose sur les mêmes réactions, les mêmes procédures et les mêmes conditions que celles décrites ci-dessus pour préparer le N,N,N',N'-(tétraméthyl)-1,6-diamino-2,5-hexanediol, mais en utilisant le 1,7-octadiène au lieu du 1,5-hexadiène en tant que précurseur pour la première réaction d'époxydation. La réaction d'époxydation produit du 1,2,7,8-diépoxyoctane utilisé dans la deuxième réaction d'addition avec la diméthylamine.

La synthèse des autres composés selon l'invention, le N,N,N',N'-tétraméthyl-1,7-diamino-2,6-heptanediol (R=r3, n=3), N,N,N',N'-tétraméthyl-1,9-diamino-2,8-nonanediol (R=r4, n=5), et N,N,N',N'-tétraméthyl-1,10-diamino-2,9-décanediol (R=r5, n=6) repose sur les mêmes réactions, les mêmes procédures et les mêmes conditions que celles décrites ci-dessus pour préparer le N,N,N',N'-(tétraméthyl)-1,6-diamino-2,5-hexanediol ou le N,N,N',N'-(tétraméthyl)-1,8-diamino-2,7-octanediol, mais en utilisant les composés figurant dans le tableau 1 suivant en tant que précurseurs et produits intermédiaires des réactions :

Tableau 1

| Nom du compose selon la formule générale (I) | Nom de l'alpha- oméga-diène | Nom du diépoxyalcane |
|---|---|---|
| N,N,N',N'-tétraméthyl-1,7-diamino-2,6-heptanediol | 1,6-heptadiène | 1,2,6,7-diépoxyheptane |
| N,N,N',N'-tétraméthyl-1,9-diamino-2,8-nonanediol | 1,8-nonadiène | 1,2,8,9-diépoxynonane |
| N,N,N',N'-tétraméthyl-1,10-diamino-2,9-décanediol | 1,9-décadiène | 1,2-9,10-diépoxydécane |

De préférence, la première réaction d'époxydation et la deuxième réaction d'addition sont conduites en deux étapes successives lors de la préparation des composés azotés selon l'invention.

Utilisation des composés selon l'invention dans le traitement d'effluents gazeux

Les composés selon l'invention peuvent être utilisés dans différents domaines de la chimie, et peuvent être avantageusement utilisés dans le domaine du traitement de gaz d'origine industrielle et du gaz naturel.

La présente invention propose d'éliminer les composés acides d'un effluent gazeux en mettant en oeuvre en solution aqueuse comprenant au moins un composé azoté selon la formule générale (I), et avantageusement le N,N,N',N'-(tétraméthyl)-1,6-diamino-2,5-hexanediol et le N,N,N',N'-(tétraméthyl)-1,8-diamino-2,7-octanediol. La solution est mise en contact avec l'effluent gazeux pour éliminer des composés acides contenus qu'il contient.

Le procédé d'élimination des composés acides contenus dans un effluent gazeux selon l'invention peut en particulier être mis en oeuvre pour l'élimination sélective de l'$H_2S$ par rapport au $CO_2$ d'un effluent gazeux comportant de l'$H_2S$ et du $CO_2$, par exemple du gaz naturel.

Le procédé d'élimination des composés acides contenus dans un effluent gazeux selon l'invention peut également être avantageusement mis en oeuvre pour le captage de $CO_2$ de gaz d'origine industrielle et du gaz naturel, par exemple de fumées de combustion.

L'utilisation des diamines tertiaires béta-hydroxylées selon l'invention permet d'obtenir de bonnes performances en terme de capacité cyclique d'absorption des gaz acides et/ou de sélectivité d'absorption vis-à-vis de l'$H_2S$, notamment une sélectivité d'absorption vis-à-vis de l'$H_2S$ plus importante que des amines de référence telles que la N-méthyldiéthanolamine (MDEA) pour une capacité cyclique d'absorption des gaz acides équivalente ou supérieure.

*Composition de la solution absorbante*

La solution absorbante mise en oeuvre pour l''élimination des composés acides contenus dans un effluent gazeux comporte:

- de l'eau;
- au moins au moins un composé azoté appartenant à la famille des diamines tertiaires répondant à la formule générale (I).

La solution absorbante peut comporter du N,N,N',N'-(tétraméthyl)-1,6-diamino-2,5-hexanediol ou du N,N,N',N'-(tétraméthyl)-1,8-diamino-2,7-octanediol, ou un mélange des deux composés.

Les amines selon la formule générale (I) peuvent être en concentration variable dans la solution absorbante, par exemple comprise entre 5 % et 95 % poids, de préférence entre 10 % et 90 % poids, encore plus préférentiellement entre 20 % et 60 % poids, et de manière très préférée entre 25 % et 50 % poids, bornes incluses.

La solution absorbante peut contenir entre 5 % et 95 % poids d'eau, de préférence entre 10 % et 90 % poids d'eau, plus préférentiellement entre 40 % et 80 % poids d'eau, et de manière très préférée de 50% à 75% d'eau, bornes incluses.

La somme des fractions massiques exprimées en % poids des différents composés de la solution absorbante est égale à 100 % en poids de la solution absorbante.

Selon un mode de réalisation, la solution absorbante peut contenir en outre au moins une amine supplémentaire qui est une amine tertiaire telle que la N-méthyldiéthanolamine, la triéthanolamine, la diéthylmonoéthanolamine, la diméthylmonoéthanolamine, ou l'éthyldiéthanolamine, ou qui est une amine secondaire ayant un encombrement stérique sévère, cet encombrement étant défini soit par la présence de deux carbones secondaires en alpha de l'azote, soit par au moins un carbone tertiaire en alpha de l'azote. On entend par ladite amine supplémentaire tout composé possédant au moins une fonction amine tertiaire ou secondaire sévèrement encombrée. La concentration de ladite amine supplémentaire tertiaire ou secondaire sévèrement encombrée dans la solution absorbante peut être comprise entre 5 % et 95 % poids, de préférence entre 5 % et 50 % poids, de manière très préférée entre 5 % et 30 % poids.

Selon un mode de réalisation, les aminés selon la formule générale (I) peuvent être formulées avec un ou plusieurs composés contenant au moins une fonction amine primaire ou secondaire. Par exemple, la solution absorbante comporte jusqu'à une concentration de 30 % poids, de préférence inférieure à 15 % poids, de préférence inférieure à 10 % poids dudit composé contenant au moins une fonction amine primaire ou secondaire. De préférence, la solution absorbante comporte au moins 0,5 % poids dudit composé contenant au moins une fonction amine primaire ou secondaire. Ledit composé permet d'accélérer la cinétique d'absorption du $CO_2$ et, dans certains cas, du COS contenu dans le gaz à traiter.

Une liste non exhaustive de composés contenant au moins une fonction amine primaire ou secondaire qui peuvent entrer dans la formulation est donnée ci-dessous :

- la monoéthanolamine ;
- la diéthanolamine ;

- la N-butyléthanolamine ;
- l'aminoéthyléthanolamine ;
- le diglycolamine ;
- la pipérazine ;
- la 1-méthylpipérazine ;
- la 2-méthylpipérazine ;
- l'homopipérazine ;
- la N-(2-hydroxyéthyl)pipérazine ;
- la N-(2-aminoéthyl)pipérazine ;
- la morpholine ;
- la 3-(méthylamino)propylamine ;
- la 1,6-hexanediamine et tous ses dérivés diversement N-alkylés tels par exemple la N,N,-diméthyl-1,6-hexanediamine, la N,N'-diméthyl-1,6-hexanediamine, la N-méthyl-1,6-hexanediamine ou la N,N',N'-triméthyl-1,6-hexanediamine.

La solution absorbante comprenant au moins un composé selon l'invention peut comprendre un mélange d'amines supplémentaires tels que définies ci-dessus.

Selon un mode de réalisation, la solution absorbante peut contenir des composés organiques non réactifs vis à vis des composés acides (couramment nommé "solvants physiques"), qui permettent d'augmenter la solubilité d'au moins un ou plusieurs composés acides de l'effluent gazeux. Par exemple, la solution absorbante peut comporter entre 5% et 50% poids de solvant physique tel que des alcools, des éthers, des étheralcools, des éthers de glycol et de polyéthylèneglycol, des thioéthers de glycol, des esters et alkoxyesters de glycol et de polyéthylèneglycol, des esters de glycérol, des lactones, des lactames, des pyrrolidones N-alkylées, des dérivés de la morpholine, de la morpholin-3-one, des imidazoles et des imidazolidinones, des pipéridones N-alkylées, des cyclotétraméthylènesulfones, des N-alkylformamides, des N-alkylacétamides, des éthers-cétones des carbonates d'alkyles ou des phosphates d'alkyles et leur dérivés.

A titre d'exemple et de façon non limitative, il peut s'agir du méthanol, de l'éthanol, du 2-éthoxyéthanol, du triéthylèneglycoldiméthyléther, du tétraéthylèneglycoldiméthyléther, du pentaéthylèneglycoldiméthyléther, de l'hexaéthylèneglycoldiméthyléther, de l'heptaéthylèneglycoldiméthyléther, de l'octaéthylèneglycoldiméthyléther, du butoxyacétate de diéthylèneglycol, du triacétate de glycérol, du sulfolane, de la N-méthylpyrrolidone, de la N-méthylmorpholin-3-one, du N,N-diméthylformamide, de la N-formyl-morpholine, de la N,N-diméthyl-imidazolidin-2-one, du N-méthylimidazole, de l'éthylèneglycol, du diéthylèneglycol, du triéthylèneglycol, ,du thiodiglycol, du carbonate de propylène, du tributylphosphate.

*<u>Nature des effluents gazeux</u>*

Les solutions absorbantes comprenant au moins un composé azoté selon l'invention peuvent être mises en oeuvre pour désacidifier les effluents gazeux suivants : le gaz naturel, les gaz de synthèse, les fumées de combustion, les gaz de raffinerie, les gaz acides issus d'une unité aux amines, les gaz issus d'une unité de réduction en queue du procédé Claus, les gaz de fermentation de biomasse, les gaz de cimenterie, les fumées d'incinérateur. Ces effluents gazeux contiennent un ou plusieurs des composés acides suivants : le $CO_2$, l'$H_2S$, des mercaptans (par exemple le méthylmercaptan ($CH_3SH$), l'éthylmercaptan ($CH_3CH_2SH$), le propylmercaptan ($CH_3CH_2CH_2SH$)), le COS, le $CS_2$, le $SO_2$.

Les fumées de combustion sont produites notamment par la combustion d'hydrocarbures, de biogaz, de charbon dans une chaudière ou pour une turbine à gaz de combustion, par exemple dans le but de produire de l'électricité. A titre d'illustration, on peut mettre en oeuvre un procédé de désacidification utilisant les composés selon l'invention pour absorber au moins 70 %, de préférence au moins 80 % voire au moins 90 % du $CO_2$ contenu dans les fumées de combustion. Ces fumées ont généralement une température comprise entre 20°C et 60°C, une pression comprise entre 1 et 5 bar et peuvent comporter entre 50 % et 80 % d'azote, entre 5 % et 40 % de dioxyde de carbone, entre 1 % et 20 % d'oxygène, et quelques impuretés comme des SOx et des NOx, s'ils n'ont pas été éliminés en amont du procédé de désacidification. En particulier, le procédé de désacidification utilisant les composés selon l'invention est particulièrement bien adapté pour absorber le $CO_2$ contenu dans des fumées de combustion comportant une faible pression partielle de $CO_2$, par exemple une pression partielle de $CO_2$ inférieure à 200 mbar.

Le procédé de désacidification utilisant les composés selon l'invention peut être mis en oeuvre pour désacidifier un gaz de synthèse. Le gaz de synthèse contient du monoxyde de carbone CO, de l'hydrogène $H_2$ (généralement dans un ratio $H_2$/CO égale à 2), de la vapeur d'eau (généralement à saturation à la température où le lavage est effectué) et du dioxyde de carbone $CO_2$ (de l'ordre de la dizaine de pourcents). La pression est généralement comprise entre 20 et 30 bar, mais peut atteindre jusqu'à 70 bar. Il peut contenir, en outre, des impuretés soufrées ($H_2S$, COS, etc.), azotées

($NH_3$, HCN) et halogénées.

Le procédé de désacidification utilisant les composés selon l'invention peut être mis en oeuvre pour désacidifier un gaz naturel. Le gaz naturel est constitué majoritairement d'hydrocarbures gazeux, mais peut contenir plusieurs des composés acides suivants : le $CO_2$, l'$H_2S$, des mercaptans, du COS, du $CS_2$. La teneur de ces composés acides est très variable et peut aller jusqu'à 70 % en volume pour le $CO_2$ et jusqu'à 40 % en volume pour l'$H_2S$. La température du gaz naturel peut être comprise entre 20°C et 100°C. La pression du gaz naturel à traiter peut être comprise entre 10 et 200 bar. L'invention peut être mise en oeuvre pour atteindre des spécifications généralement imposées sur le gaz désacidifié, qui sont moins de 2 % de $CO_2$, voire moins de 50 ppm de $CO_2$ pour réaliser ensuite une liquéfaction du gaz naturel et moins de 4 ppm d'$H_2S$, et moins de 50 ppm, voire moins de 10 ppm, volume de soufre total.

*Procédé d'élimination des composés acides dans un effluent gazeux*

La mise en oeuvre d'une solution aqueuse comportant un composé selon la formule générale (I) pour désacidifier un effluent gazeux est réalisée de façon schématique en effectuant une étape d'absorption suivie d'une étape de régénération, par exemple tel que représenté par la figure 1.

En référence à la figure 1, l'installation de désacidification d'un effluent gazeux selon l'invention comprend une colonne d'absorption C1 munie de moyens de mise en contact entre gaz et liquide, par exemple un garnissage vrac, un garnissage structuré ou des plateaux. L'effluent gazeux à traiter est acheminé par une conduite 1 débouchant au fond de la colonne C1. Une conduite 4 permet l'introduction de la solution absorbante en tête de la colonne C1. Une conduite 2 permet l'évacuation du gaz traité (désacidifié), et une conduite 3 permet d'acheminer la solution absorbante enrichie en composés acides suite à l'absorption vers une colonne de régénération C2. Cette colonne de régénération C2 est équipée d'internes de mise en contact entre gaz et liquide, par exemple des plateaux, des garnissages en vrac ou structurés. Le fond de la colonne C2 est équipée d'un rebouilleur R1 qui apporte la chaleur nécessaire à la régénération en vaporisant une fraction de la solution absorbante. La solution enrichie en composés acides est introduite en tête de la colonne de régénération C2 par une conduite 5. Une conduite 7 permet d'évacuer au sommet de la colonne C2 le gaz enrichi en composés acides libérés lors de la régénération, et une conduite 6 disposée au fond de la colonne C2 permet d'envoyer la solution absorbante régénérée vers la colonne d'absorption C1. Un échangeur de chaleur E1 permet de récupérer la chaleur de la solution absorbante régénérée issue de la colonne C2 pour chauffer la solution absorbante enrichie en composés acides sortant de la colonne d'absorption C1.

L'étape d'absorption consiste à mettre en contact l'effluent gazeux arrivant par la conduite 1 avec la solution absorbante arrivant par la conduite 4. Lors du contact, les fonctions amines des molécules selon la formule générale (I) de la solution absorbante réagissent avec les composés acides contenus dans l'effluent de manière à obtenir un effluent gazeux appauvri en composés acides qui est évacué par la conduite 2 en tête de la colonne C1 et une solution absorbante enrichie en composés acides évacuée par la conduite 3 en fond de la colonne C1 pour être régénérée.

L'étape d'absorption des composés acides peut être réalisée à une pression dans la colonne C1 comprise entre 1 bar et 200 bar, de préférence entre 1 bar et 120 bar, plus préférentiellement entre 20 bar et 100 bar pour le traitement d'un gaz naturel, de préférence entre 1 bar et 3 bar pour le traitement des fumées industrielles, et à une température dans la colonne C1 comprise entre 20°C et 100°C, préférentiellement comprise entre 30°C et 90°C, voire entre 30 et 60°C.

L'étape de régénération consiste notamment à chauffer et, éventuellement à détendre, la solution absorbante enrichie en composés acides afin de libérer les composés acides sous forme gazeuse. La solution absorbante enrichie en composés acides sortant de la colonne C1 est introduite dans l'échangeur de chaleur E1, où elle est réchauffée par le flux circulant dans la conduite 6 en provenance de la colonne de régénération C2. La solution réchauffée en sortie de E1 est introduite dans la colonne de régénération C2 par la conduite 5.

Dans la colonne de régénération C2, sous l'effet de la mise en contact de la solution absorbante arrivant par la conduite 5 avec la vapeur produite par le rebouilleur, les composés acides sont libérés sous forme gazeuse et évacués en tête de colonne C2 par la conduite 7. La solution absorbante régénérée, c'est-à-dire appauvrie en composés acides, est évacuée par la conduite 6 et est refroidie dans E1, puis recyclée dans la colonne d'absorption C1 par la conduite 4.

L'étape de régénération peut être réalisée par régénération thermique, éventuellement complétée par une ou plusieurs étapes de détente. Par exemple, la solution absorbante enrichie en composés acides évacuée par la conduite 3 peut être envoyée dans un premier ballon de détente (non représenté), avant son passage dans l'échangeur de chaleur E1. Dans le cas d'un gaz naturel, la détente permet d'obtenir un gaz évacué au sommet du ballon contenant la majeure partie des hydrocarbures aliphatiques co-absorbés par la solution absorbante. Ce gaz peut éventuellement être lavé par une fraction de la solution absorbante régénérée et le gaz ainsi obtenu peut être utilisé comme gaz combustible. Le ballon de détente opère de préférence à une pression inférieure à celle de la colonne d'absorption C1 et supérieure à celle de la colonne de régénération C2. Cette pression est généralement fixée par les conditions d'utilisation du gaz combustible, et est typiquement de l'ordre de 5 à 15 bar. Le ballon de détente opère à une température sensiblement identique à celle de la solution absorbante obtenue en fond de la colonne d'absorption C1.

La régénération peut être effectuée à une pression dans la colonne C2 comprise entre 1 bar et 5 bar, voire jusqu'à 10 bar et à une température dans la colonne C2 comprise entre 100°C et 180°C, de préférence comprise entre 110°C et 170°C, plus préférentiellement entre 120°C et 140°C. De manière préférée, la température de régénération dans la colonne C2 est comprise entre 155°C et 180°C dans le cas où l'on souhaite réinjecter les gaz acides. De manière préférée, la température de régénération dans la colonne C2 est comprise entre 115°C et 130°C dans les cas où le gaz acide est envoyé à l'atmosphère ou dans un procédé de traitement aval, comme un procédé Claus ou un procédé de traitement de gaz de queue.

## Exemples

Les exemples ci-dessous illustrent, de manière non limitative, la synthèse des composés selon l'invention, ainsi que certaines des performances de ces composés lorsqu'ils sont utilisés en solution aqueuse pour éliminer des composés acides, comme le $CO_2$ ou l'$H_2S$, contenus dans un effluent gazeux par mise en contact de l'effluent gazeux avec la solution.

Exemple 1 : synthèse des molécules selon l'invention.

Les exemples suivants illustrent la synthèse des composés azotés selon l'invention, étant entendu que toutes les possibilités de synthèse de ces molécules tant au niveau des routes de synthèses que des modes opératoires possibles ne sont pas ici décrites.

*Exemple de synthèse du N,N,N',N'-(tétraméthyl)-1,6-diamino-2,5-hexanediol*

A une solution de 75,0 g de 1,5-hexadiène dans 1200 ml de dichlorométhane maintenue entre 0°C et 5°C, on introduit en quatre heures, par petites fractions, 460,9 g (2,67 moles) d'acide 3-chloroperbenzoïque. Après retour à la température ambiante, on procède à la filtration du milieu. Le filtrat est lavé deux fois avec 750 ml d'une solution aqueuse de sulfite de sodium à 10% puis avec 750 ml d'eau. Après distillation, on obtient 86,1g d'un produit dont le spectre RMN-$^{13}C$ ($CDCl_3$), caractérisé par les données ci-dessous, est conforme à celui du 1,2,5,6-diépoxyhexane.

46,1 ppm : [$\mathbf{C}H_2$(O)CH]-$CH_2$-$CH_2$-[CH(O)$CH_2$]
50,8 ppm : [$CH_2$(O)**C**H]-$CH_2$-$CH_2$-[CH(O)$CH_2$]
28,6 ppm : [$CH_2$(O)CH]-$\mathbf{C}H_2$-$CH_2$-[CH(O)$CH_2$]
28,2 ppm : [$CH_2$(O)CH]-$CH_2$-$\mathbf{C}H_2$-[CH(O)$CH_2$]
51,0 ppm : [$CH_2$(O)CH]-$CH_2$-$CH_2$-[**C**H(O)$CH_2$]
46,1 ppm : [$CH_2$(O)CH]-$CH_2$-$CH_2$-[CH(O)$\mathbf{C}H_2$]

A 1636,0 g d'une solution aqueuse de diméthylamine à 40%, on introduit en deux heures en maintenant la température à 5°C, 85,0 g (0,74 mole) de 1,2,5,6-diépoxyhexane. Après retour à la température ambiante, l'excès de diméthylamine ainsi que l'eau sont éliminés. Après distillation sous pression réduite, on isole 115,0 g d'un produit dont le spectre RMN-$^{13}C$ ($CDCl_3$), caractérisé par les données ci-dessous, est conforme à celui du N,N,N',N'-(tétraméthyl)-1,6-diamino-2,5-hexanediol.

45,3 ppm : ($\mathbf{C}H_3$)$_2$N-$CH_2$-CH(OH)-$CH_2$-$CH_2$-CH(OH)-$CH_2$-N($CH_3$)$_2$
30,8 ppm : ($CH_3$)$_2$N-$\mathbf{C}H_2$-CH(OH)-$CH_2$-$CH_2$-CH(OH)-$CH_2$-N($CH_3$)$_2$
67,0 ppm : ($CH_3$)$_2$N-$CH_2$-**C**H(OH)-$CH_2$-$CH_2$-CH(OH)-$CH_2$-N($CH_3$)$_2$
65,3 ppm : ($CH_3$)$_2$N-$CH_2$-CH(OH)-$\mathbf{C}H_2$-$CH_2$-CH(OH)-$CH_2$-N($CH_3$)$_2$
65,3 ppm : ($CH_3$)$_2$N-$CH_2$-CH(OH)-$CH_2$-$\mathbf{C}H_2$-CH(OH)-$CH_2$-N($CH_3$)$_2$
66,7 ppm : ($CH_3$)$_2$N-$CH_2$-CH(OH)-$CH_2$-$CH_2$-**C**H(OH)-$CH_2$-N($CH_3$)$_2$
31,1 ppm : ($CH_3$)$_2$N-$CH_2$-CH(OH)-$CH_2$-$CH_2$-CH(OH)-$\mathbf{C}H_2$-N($CH_3$)$_2$
45,3 ppm : ($CH_3$)$_2$N-$CH_2$-CH(OH)-$CH_2$-$CH_2$-CH(OH)-$CH_2$-N($\mathbf{C}H_3$)$_2$

*Exemple de synthèse du N,N,N',N'-(tétraméthyl)-1,8-diamino-2,7-octanediol*

A une solution de 100,0 g de 1,7-octadiène dans 1200 ml de dichlorométhane maintenue entre 0°C et 5°C, on introduit en quatre heures, par petites fractions, 457,5 g (2,65 moles) d'acide 3-chloroperbenzoique. Après retour à la température ambiante, on procède à la filtration du milieu. Le filtrat est lavé avec deux fois avec 750 ml d'une solution aqueuse de sulfite de sodium à 10%, puis avec 750 ml d'eau . Après distillation, on obtient 110,0 g d'un produit dont le spectre RMN-$^{13}C$ ($CDCl_3$), caractérisé par les données ci-dessous, est conforme à celui du 1,2,7,8-diépoxyoctane.

45,6 ppm : [**C**H2(O)CH]-CH2-CH2-CH2-CH2-[CH(O)CH2]
50,8 ppm : [CH2(O)**C**H]-CH2-CH2-CH2-CH2-[CH(O)CH2]
31,4 ppm : [CH2(O)CH]-**C**H2-CH2-CH2-CH2-[CH(O)CH2]
24,9 ppm ; [CH2(O)CH]-CH2-**C**H2-CH2-CH2-[CH(O)CH2]
24,9 ppm : [CH2(O)CH]-CH2-CH2-**C**H2-CH2-[CH(O)CH2]
31,4 ppm : [CH2(O)CH]-CH2-CH2-CH2-**C**H2-[CH(O)CH2]
50,8 ppm : [CH2(O)CH]-CH2-CH2-CH2-CH2-[**C**H(O)CH2]
45,6 ppm : [CH2(O)CH]-CH2-CH2-CH2-CH2-[CH(O)**C**H2]

A 769,0 g d'une solution aqueuse de diméthylamine à 40 %, on introduit en deux heures en maintenant la température à 5°C, 100,0 g (0,70 mole) de 1,2,7,8-diépoxyoctane. Après retour à la température ambiante, l'excès de diméthylamine ainsi que l'eau sont éliminés. Après distillation sous pression réduite, on isole 143,0 g d'un produit dont le spectre RMN-$^{13}C$ ($CDCl_3$), caractérisé par les données ci-dessous, est conforme à celui du N,N,N',N'-(tétraméthyl)-1,8-diamino-2,7-octanediol.

45,3 ppm : (**C**H3)2N-CH2-CH(OH)-CH2-CH2-CH2-CH2-CH(OH)-CH2-N(CH3)2
65,4 ppm : (CH3)2N-**C**H2-CH(OH)-CH2-CH2-CH2-CH2-CH(OH)-CH2-N(CH3)2
66,7 ppm : (CH3)2N-CH2-**C**H(OH)-CH2-CH2-CH2-CH2-CH(OH)-CH2-N(CH3)2
34,7 ppm : (CH3)2N-CH2-CH(OH)-**C**H2-CH2-CH2-CH2-CH(OH)-CH2-N(CH3)2
25,7 ppm : (CH3)2N-CH2-CH(OH)-CH2-**C**H2-CH2-CH2-CH(OH)-CH2-N(CH3)2
25,7 ppm ; (CH3)2N-CH2-CH(OH)-CH2-CH2-**C**H2-CH2-CH(OH)-CH2-N(CH3)2
34,7 ppm : (CH3)2N-CH2-CH(OH)-CH2-CH2-CH2-**C**H2-CH(OH)-CH2-N(CH3)2
66,7 ppm : (CH3)2N-CH2-CH(OH)-CH2-CH2-CH2-CH2-**C**H(OH)-CH2-N(CH3)2
65,4 ppm : (CH3)2N-CH2-CH(OH)-CH2-CH2-CH2-CH2-CH(OH)-**C**H2-N(CH3)2
45,3 ppm : (CH3)2N-CH2-CH(OH)-CH2-CH2-CH2-CH2-CH(OH)-CH2-N(**C**H3)2

Exemple 2 : Vitesse d'absorption du $CO_2$ d'une formulation d'amine pour un procédé d'absorption sélective.

On effectue des essais comparatifs d'absorption du $CO_2$ par différentes solutions absorbantes :

- une solution absorbante comprenant du N,N,N',N'-(tétraméthyl)-1,6-diamino-2,5-hexanediol selon l'invention à 49% poids dans l'eau ;
- une solution aqueuse de N-méthyldiéthanolamine (MDEA) à 47 % en poids de MDEA, qui constitue une solution absorbante de référence pour une élimination sélective en traitement de gaz ;
- une solution aqueuse de 1,2-bis-(pyrrolidinyléthoxy)-éthane à 50 % en poids de 1,2-bis-(pyrrolidinyléthoxy)-éthane, qui est un diaminoéther avec deux fonctions amines tertiaires selon la formule générale du brevet US 4,405,582, mais qui ne possède pas de fonction alcool et qui n'entre pas dans la formule générale (I) selon l'invention ;
- une solution aqueuse de 1,2-bis-(tertiobutylaminoéthoxy)-éthane à 40 % en poids de 1,2-bis-(tertiobutylaminoéthoxy)-éthane, qui est un diaminoéther avec deux fonctions secondaires ayant un encombrement stérique sévère des atomes d'azote selon la formule générale du brevet US 4,405,583, sans fonction alcool et qui n'entre pas dans la formule générale (I) selon l'invention ;
- une solution aqueuse de N,N,N',N'-tétraméthyl-1,6-hexanediamine (TMHDA) à 50 % en poids de TMHDA, qui est une diamine tertiaire divulguée dans le brevet FR2934172, mais qui ne possède pas de fonction alcool et qui n'entre pas dans la formule générale (I) selon l'invention.

Pour chaque essai, on mesure le flux d'absorption du $CO_2$ par la solution absorbante aqueuse dans un réacteur fermé, du type cellule de Lewis. 200 g de solution est introduite dans le réacteur fermé, régulé à une température de 50°C . On réalise quatre injections successives de $CO_2$ de 100 à 200 mbar dans la phase vapeur du réacteur ayant un volume de 200 $cm^3$. La phase gaz et la phase liquide sont agitées à 100 tours/minutes et entièrement caractérisées du point de vue hydrodynamique. Pour chaque injection, on mesure la vitesse d'absorption du $CO_2$ par variation de pression dans la phase gaz. On détermine ainsi un coefficient de transfert global Kg par une moyenne des résultats obtenus sur les quatre injections.

Les résultats obtenus sont présentés dans le tableau 2 ci-dessous en vitesse d'absorption relative par rapport à la solution absorbante aqueuse de référence comprenant 47 % poids de MDEA, cette vitesse d'absorption relative étant définie par le rapport du coefficient de transfert global de la solution absorbante testée sur le coefficient de transfert global de la solution absorbante de référence (avec MDEA).

Tableau 2

| Composé | Concentration (% en poids) | Vitesse d'absorption relative du $CO_2$ à 50°C |
|---|---|---|
| MDEA | 47 | 1,00 |
| 1,2-bis-(pyrrolidinyléthoxy)-éthane (selon le brevet US 4,405,582) | 50 | 1,43 |
| 1,2-bis-(tertiobutylaminoéthoxy)-éthane (selon le brevet US 4,405,583) | 40 | 1,74 |
| TMHDA (selon FR2934172) | 50 | 2,72 |
| N,N,N',N'-(tétraméthyl)-1,6-diamino-2,5-hexanediol | 49 | 0,87 |

L'examen des résultats fait ressortir, dans ces conditions de test, une vitesse d'absorption du $CO_2$ par la solution absorbante selon l'invention plus lente par rapport à la formulation de référence avec la MDEA, et par rapport aux solutions absorbantes avec certaines molécules de l'art antérieur. Il apparaît donc que le composé exemplifié selon l'invention présente étonnamment un intérêt particulier et amélioré dans le cas d'une désacidification sélective d'un effluent gazeux dans laquelle on cherche à limiter la cinétique d'absorption du $CO_2$.

Exemple 3 : Capacité d'absorption de l'$H_2S$ d'une formulation de N,N,N',N'-(tétraméthyl)-1,6-diamino-2,5-hexanediol pour un procédé de traitement de gaz acide.

Les performances de capacité d'absorption de l'$H_2S$ à 40 °C d'une solution aqueuse de N,N,N',N'-(tétraméthyl)-1,6-diamino-2,5-hexanediol selon l'invention, contenant 49 % en poids de N,N,N',N'-(tétraméthyl)-1,6-diamino-2,5-hexanediol est comparée à celles d'une solution aqueuse de MDEA contenant 50 % en poids de MDEA, qui constitue une solution absorbante de référence pour la désacidification de gaz contenant de l'$H_2S$.

On réalise un test d'absorption à 40 °C sur des solutions aqueuses d'amine au sein d'une cellule d'équilibre thermostatée. Ce test consiste à injecter dans la cellule d'équilibre, préalablement remplie de solution aqueuse d'amine dégazée, une quantité connue de gaz acide, de l'$H_2S$ dans cet exemple, puis à attendre l'établissement de l'état d'équilibre. Les quantités de gaz acide absorbées dans la solution aqueuse d'amine sont alors déduites des mesures de température et de pression grâce à des bilans de matière et de volume. Les solubilités sont représentées de manière classique sous la forme des pressions partielles en $H_2S$ (en bar) en fonction du taux de charge en $H_2S$ (en mol d' $H_2S$/kg de solution absorbante et en mol d' $H_2S$/mol d'amine).

Dans le cas d'une désacidification en traitement de gaz naturel, les pressions partielles en $H_2S$ rencontrées dans les gaz acides sont typiquement comprises entre 0,1 et 1 bar, à une température de 40°C. A titre d'exemple, dans cette gamme industrielle, on compare dans le tableau 3 ci-dessous les taux de charge d'$H_2S$ obtenus à 40°C pour différentes pressions partielles en $H_2S$ entre la solution absorbante de MDEA à 50 % poids et la solution absorbante de N,N,N',N'-(tétraméthyl)-1,6-diamino-2,5-hexanediol à 49 % poids.

Tableau 3

| | Solution aqueuse de N,N,N',N'-(tétraméthyl)-1,6-diamino-2,5-hexanediol à 49 % poids à 40 °C | | Solution aqueuse de MDEA à 50 % poids à 40 °C | |
|---|---|---|---|---|
| Pression partielle en $H_2S$ (bar) | Taux de charge en $H_2S$ (mol/mol d'amine) | Taux de charge en $H_2S$ (mol/kg) | Taux de charge en $H_2S$ (mol/mol d'amine) | Taux de charge en $H_2S$ (mol/kg) |
| 0,10 | 0,94 | 2,26 | 0,21 | 0,88 |
| 1,00 | 1,75 | 4,20 | 0,69 | 2,95 |

A 40 °C, quelle que soit la pression partielle en $H_2S$, la capacité d'absorption de la solution aqueuses de N,N,N',N'-(tétraméthyl)-1,6-diamino-2,5-hexanediol selon l'invention est supérieure à celle de la solution de MDEA. A une pression partielle en $H_2S$ de 0,10 bar, l'écart entre les taux de charge en $H_2S$ des deux solutions absorbantes s'élève à 1,38 mol/kg avec une capacité d'absorption pour la solution absorbante de N,N,N',N'-(tétraméthyl)-1,6-diamino-2,5-hexanediol augmentée de 157% par rapport à la solution absorbante de MDEA de référence . A une pression partielle

en $H_2S$ de 1 bar, l'augmentation de taux de charge en $H_2S$ pour la solution absorbante de N,N,N',N'-(tétraméthyl)-1,6-diamino-2,5-hexanediol est encore de 42% par rapport à la solution absorbante de MDEA de référence. On constate donc que la solution aqueuse de N,N,N',N'-(tétraméthyl)-1,6-diamino-2,5-hexanediol à 49 % poids a une capacité d'absorption de l'$H_2S$ plus élevée que la solution aqueuse de MDEA de référence à 50 % poids de MDEA à 40°C, dans la gamme de pressions partielles en $H_2S$ comprise entre 0,1 et 1 bar correspondant à une gamme de pression partielle représentative des conditions industrielles usuelles.

Il apparaît donc que cette molécule exemplifiée selon l'invention permet de réduire les débits de solution absorbante à mettre en oeuvre sur des applications de désacidification de gaz contenant $H_2S$ par rapport à la solution absorbante de MDEA de référence.

L'absorption du $CO_2$ étant plus lente dans une solution aqueuse de N,N,N',N'-(tétraméthyl)-1,6-diamino-2,5-hexanediol que dans une solution aqueuse de MDEA (voir exemple 2 ci-dessus) et la capacité d'absorption équivalente ou supérieure en gaz acides, notamment en $H_2S$, de la solution absorbante de N,N,N',N'-(tétraméthyl)-1,6-diamino-2,5-hexanediol par rapport à une solution aqueuse de MDEA comme illustré dans le présent exemple, il apparaît que cette molécule exemplifiée selon l'invention permet de réduire les débits de solution absorbante à mettre en oeuvre sur des applications de désacidification sélective ($H_2S$ par rapport au $CO_2$) pour absorber un débit donné de $H_2S$ tout en réduisant le débit de $CO_2$ co-absorbé par rapport à la solution absorbante de MDEA de référence.

Exemple 4 : Capacité d'absorption de l'$H_2S$ d'une formulation de N,N,N',N'-(tétraméthyl)-1,8-diamino-2,7-octanediol pour un procédé de traitement de gaz acide.

Les performances de capacité d'absorption de l'$H_2S$ à 40 °C d'une solution aqueuse de N,N,N',N'-(tétraméthyl)-1,8-diamino-2,7-octanediol selon l'invention, contenant 50% en poids de N,N,N',N'-(tétraméthyl)-1,8-diamino-2,7-octanediol sont comparées à celles d'une solution aqueuse de MDEA contenant 50 % en poids de MDEA, qui constitue une solution absorbante de référence pour la désacidification de gaz contenant de l'$H_2S$.

On réalise un test d'absorption à 40 °C selon le mode opératoire décrit dans l'exemple précédent.

Dans le cas d'une désacidification en traitement de gaz naturel, les pressions partielles en $H_2S$ rencontrées dans les gaz acides sont typiquement comprises entre 0,1 et 1 bar, à une température de 40°C. A titre d'exemple, dans cette gamme industrielle, on compare dans le tableau 4 ci-dessous les taux de charge d'$H_2S$ obtenus à 40°C pour différentes pressions partielles en $H_2S$ entre la solution absorbante de MDEA à 50 % poids et la solution absorbante N,N,N',N'-(tétraméthyl)-1,8-diamino-2,7-octanediol à 50 % poids.

Tableau 4

| | Solution aqueuse de N,N,N',N'-(tétraméthyl)-1,8-diamino-2,7-octanediol à 50 % poids à 40 °C | | Solution aqueuse de MDEA à 50 % poids à 40 °C | |
|---|---|---|---|---|
| Pression partielle en $H_2S$ (bar) | Taux de charge en $H_2S$ (mol/mol d'amine) | Taux de charge en $H_2S$ (mol/kg) | Taux de charge en $H_2S$ (mol/mol d'amine) | Taux de charge en $H_2S$ (mol/kg) |
| 0,10 | 1,04 | 2,25 | 0,21 | 0,88 |
| 1,00 | 1,82 | 3,92 | 0,69 | 2,95 |

A 40 °C, quelle que soit la pression partielle en $H_2S$, la capacité d'absorption de la solution aqueuses de N,N,N',N'-(tétraméthyl)-1,8-diamino-2,7-octanediol selon l'invention est supérieure à celle de la solution de MDEA. A une pression partielle en $H_2S$ de 0,10 bar, l'écart entre les taux de charge en $H_2S$ des deux solutions absorbantes s'élève à 1,37 mol/kg avec une capacité d'absorption pour la solution absorbante de N,N,N',N'-(tétraméthyl)-1,8-diamino-2,7-octanediol augmentée de 156% par rapport à la solution absorbante de MDEA de référence. A une pression partielle en $H_2S$ de 1 bar, l'augmentation de taux de charge en $H_2S$ pour la solution absorbante de N,N,N',N'-(tétraméthyl)-1,8-diamino-2,7-octanediol est encore de 33% par rapport à la solution absorbante de MDEA de référence. On constate donc que la solution aqueuse de N,N,N',N'-(tétraméthyl)-1,8-diamino-2,7-octanediol à 50 % poids a une capacité d'absorption de l'$H_2S$ plus élevée que la solution aqueuse de MDEA de référence à 50 % poids de MDEA à 40°C, dans la gamme de pressions partielles en $H_2S$ comprise entre 0,1 et 1 bar correspondant à une gamme de pression partielle représentative des conditions industrielles usuelles.

Il apparaît donc que cette molécule exemplifiée selon l'invention permet de réduire les débits de solution absorbante à mettre en oeuvre sur des applications de désacidification de gaz contenant $H_2S$ par rapport à la solution absorbante de MDEA de référence.

Exemple 5 : Capacité d'absorption du $CO_2$ d'une formulation de N,N,N',N'-(tétraméthyl)-1,6-diamino-2,5-hexanediol pour un procédé de traitement de gaz acide.

Les performances de capacité d'absorption du $CO_2$ à 40 °C d'une solution aqueuse de N,N,N',N'-(tétraméthyl)-1,6-diamino-2,5-hexanediol selon l'invention, contenant 49 % en poids de N,N,N',N'-(tétraméthyl)-1,6-diamino-2,5-hexanediol est comparée à celles d'une solution aqueuse de MDEA contenant 47 % en poids de MDEA, qui constitue une solution absorbante de référence pour la désacidification de gaz contenant du $CO_2$.

On réalise un test d'absorption à 40 °C selon le mode opératoire décrit dans les exemples précédents, le gaz acide étant du $CO_2$ au lieu de l'$H_2S$.

Dans le cas d'une désacidification en traitement de gaz naturel, les pressions partielles en $CO_2$ rencontrées dans les gaz acides sont typiquement comprises entre 0,1 et 1 bar, à une température de 40°C. A titre d'exemple, dans cette gamme industrielle, on compare dans le tableau 5 ci-dessous les taux de charge en $CO_2$ obtenus à 40°C pour différentes pressions partielles en $CO_2$ entre la solution absorbante de MDEA à 47 % poids et la solution absorbante de N,N,N',N'-(tétraméthyl)-1,6-diamino-2,5-hexanediol à 49 % poids.

Tableau 5

| | Solution aqueuse de N,N,N',N'-(tétraméthyl)-1,6-diamino-2,5-hexanediol à 49 % poids à 40 °C | | Solution aqueuse de MDEA à 47 % poids à 40 °C | |
|---|---|---|---|---|
| Pression partielle en $CO_2$ (bar) | Taux de charge en $CO_2$ (mol/mol d'amine) | Taux de charge en $CO_2$ (mol/kg) | Taux de charge en $CO_2$ (mol/mol d'amine) | Taux de charge en $CO_2$ (mol/kg) |
| 0,10 | 0,88 | 2,12 | 0,22 | 0,88 |
| 1,00 | 1,65 | 3,95 | 0,69 | 2,74 |

A 40 °C, quelle que soit la pression partielle en $CO_2$, la capacité d'absorption de la solution aqueuses de N,N,N',N'-(tétraméthyl)-1,6-diamino-2,5-hexanediol selon l'invention est supérieure à celle de la solution de MDEA. A une pression partielle en $CO_2$ de 0,10 bar, l'écart entre les taux de charge en $CO_2$ des deux solutions absorbantes s'élève à 1,24 mol/kg avec une capacité d'absorption pour la solution absorbante de N,N,N',N'-(tétraméthyl)-1,6-diamino-2,5-hexanediol augmentée de 141% par rapport à la solution absorbante de MDEA de référence . A une pression partielle en $CO_2$ de 1 bar, l'augmentation de taux de charge en $CO_2$ pour la solution absorbante de N,N,N',N'-(tétraméthyl)-1,6-diamino-2,5-hexanediol est encore de 44% par rapport à la solution absorbante de MDEA de référence. On constate donc que la solution aqueuse de N,N,N',N'-(tétraméthyl)-1,6-diamino-2,5-hexanediol à 49 % poids a une capacité d'absorption du $CO_2$ plus élevée que la solution aqueuse de MDEA de référence à 47 % poids de MDEA à 40°C, dans la gamme de pressions partielles en $CO_2$ comprise entre 0,1 et 1 bar correspondant à une gamme de pression partielle représentative des conditions industrielles usuelles.

Il apparaît donc que cette molécule exemplifiée selon l'invention permet de réduire les débits de solution absorbante à mettre en oeuvre sur des applications de désacidification de gaz contenant du $CO_2$ par rapport à la solution absorbante de MDEA de référence.

Exemple 6 : Capacité d'absorption du $CO_2$ d'une formulation de N,N,N',N'-(tétraméthyl)-1,8-diamino-2.7-octanediol pour un procédé de traitement de gaz acide.

Les performances de capacité d'absorption du $CO_2$ à 40 °C d'une solution aqueuse de N,N,N',N'-(tétraméthyl)-1,8-diamino-2,7-octanediol selon l'invention, contenant 47 % en poids de N,N,N',N'-(tétraméthyl)-1,8-diamino-2,7-octanediol est comparée à celles d'une solution aqueuse de MDEA contenant 47 % en poids de MDEA, qui constitue une solution absorbante de référence pour la désacidification de gaz contenant du $CO_2$.

On réalise un test d'absorption à 40 °C selon le mode opératoire décrit dans les exemples précédents, le gaz acide étant du $CO_2$.

Dans le cas d'une désacidification en traitement de gaz naturel, les pressions partielles en $CO_2$ rencontrées dans les gaz acides sont typiquement comprises entre 0,1 et 1 bar, à une température de 40°C. A titre d'exemple, dans cette gamme industrielle, on compare dans le tableau 6 ci-dessous les taux de charge en $CO_2$ obtenus à 40°C pour différentes pressions partielles en $CO_2$ entre la solution absorbante de MDEA à 47 % poids et la solution absorbante de N,N,N',N'-(tétraméthyl)-1,8-diamino-2,7-octanediol à 47 % poids.

Tableau 6

| | Solution aqueuse de N,N,N',N'-(tétraméthyl)-1,8-diamino-2,7-octanediol à 47 % poids à 40 °C | | Solution aqueuse de MDEA à 47 % poids à 40 °C | |
|---|---|---|---|---|
| Pression partielle en $CO_2$ (bar) | Taux de charge en $CO_2$ (mol/mol d'amine) | Taux de charge en $CO_2$ (mol/kg) | Taux de charge en $CO_2$ (mol/mol d'amine) | Taux de charge en $CO_2$ (mol/kg) |
| 0,10 | 1,02 | 2,06 | 0,22 | 0,88 |
| 1,00 | 1,82 | 3,68 | 0,69 | 2,74 |

A 40 °C, quelle que soit la pression partielle en $CO_2$, la capacité d'absorption de la solution aqueuses de N,N,N',N'-(tétraméthyl)-1,8-diamino-2,7-octanediol selon l'invention est supérieure à celle de la solution de MDEA. A une pression partielle en $CO_2$ de 0,10 bar, l'écart entre les taux de charge en $CO_2$ des deux solutions absorbantes s'élève à 1,18 mol/kg avec une capacité d'absorption pour la solution absorbante de N,N,N',N'-(tétraméthyl)-1,8-diamino-2,7-octanediol augmentée de 134% par rapport à la solution absorbante de MDEA de référence. A une pression partielle en $CO_2$ de 1 bar, l'augmentation de taux de charge en $CO_2$ pour la solution absorbante de N,N,N',N'-(tétraméthyl)-1,8-diamino-2,7-hexanediol est encore de 34% par rapport à la solution absorbante de MDEA de référence. On constate donc que la solution aqueuse de N,N,N',N'-(tétraméthyl)-1,8-diamino-2,7-octanediol à 47 % poids a une capacité d'absorption du $CO_2$ plus élevée que la solution aqueuse de MDEA de référence à 47 % poids de MDEA à 40°C, dans la gamme de pressions partielles en $CO_2$ comprise entre 0,1 et 1 bar correspondant à une gamme de pression partielle représentative des conditions industrielles usuelles.

Il apparaît donc que cette molécule exemplifiée selon l'invention permet de réduire les débits de solution absorbante à mettre en oeuvre sur des applications de désacidification de gaz contenant du $CO_2$ par rapport à la solution absorbante de MDEA de référence.

Exemple 7 : Capacité de captage de solutions aqueuses de N,N,N',N'-(tétraméthyl)-1,6-diamino-2,5-hexanediol et de N,N,N',N'-(tétraméthyl)-1,8-diamino-2,7-octanediol. Application au traitement des fumées en post-combustion

Les performances de capacité de captage du $CO_2$ du N,N,N',N'-(tétraméthyl)-1,6-diamino-2,5-hexanediol et du N,N,N',N'-(tétraméthyl)-1,8-diamino-2,7-octanediol selon l'invention sont comparées notamment à celles d'une solution aqueuse de MonoEthanolAmine (MEA) à 30 % poids, qui constitue un solvant de référence pour une application de captage du $CO_2$ contenu dans des fumées en post-combustion. On réalise un test d'absorption sur des solutions aqueuses d'amine selon le mode opératoire décrit précédemment.

A titre d'exemple, on compare dans le tableau 7 les taux de charge ($\alpha$ = nb de mole gaz acide/nb de mole amine) obtenus à 40°C pour différentes pressions partielles de $CO_2$ entre une solution aqueuse de N,N,N',N'-(tétraméthyl)-1,6-diamino-2,5-hexanediol à 49% poids selon l'invention, une solution aqueuse de N,N,N',N'-(tétraméthyl)-1,8-diamino-2,7-octanediol à 47% poids selon l'invention et une solution aqueuse de MonoEthanolAmine, à 30 % en poids pour une application de captage du $CO_2$ en post-combustion.

Pour passer d'une grandeur du taux de charge obtenu au laboratoire à une grandeur caractéristique du procédé, quelques calculs sont nécessaires, et sont explicités ci-dessous pour l'application visée.

Dans le cas d'une application de captage du $CO_2$ en post-combustion, les pressions partielles de $CO_2$ dans l'effluent à traiter sont typiquement 0,1 bar avec une température de 40°C, et l'on souhaite abattre 90 % du gaz acide. On calcule la capacité cyclique $\Delta\alpha$PC exprimée en moles de $CO_2$ par kg de solvant, en considérant que le solvant atteint un taux de charge en fond de colonne d'absorption correspondant à une pression partielle d'équilibre à 40°C égale à 50% de la pression partielle dans l'effluent à traiter, soit $\alpha PPCO_2$=0,05 bar et doit au moins être régénéré à un taux de charge correspondant à une pression partielle d'équilibre à 40°C égale à 50 % de la pression dans les conditions de la tête de colonne, soit $\alpha PPCO_2$=0,005 bar pour réaliser 90 % d'abattement du $CO_2$.

$$\Delta\alpha_{PC} = \left(\alpha_{PPCO2=0,05bar} - \alpha_{PPCO2=0,005bar}\right)\cdot[A]\cdot 10/M$$

où [A] est la concentration d'amine exprimée en % poids, et M la masse molaire de l'amine en g/mol, $\alpha_{PPCO2=0,05bar}$ et $\alpha_{PPCO2=0,005bar}$ sont les taux de charge (mole $CO_2$/mole d'amine) du solvant en équilibre respectivement avec une pression partielle de 0,05 bar et 0,005 bar de $CO_2$.

L'enthalpie de réaction peut être obtenue par calcul à partir de plusieurs isothermes d'absorption du $CO_2$ en

appliquant la loi de Van't Hoff.

Tableau 7

| | | | Taux de charge $\alpha = n_{CO2}/n_{amine}$ | | | |
|---|---|---|---|---|---|---|
| Nom Générique | Concentration | T (°C) | $P_{PCO2}$ = 0,05 bar | $P_{PCO2}$ = 0,005 bar | $\Delta\alpha_{PC}$ ($mol_{CO2}$/kg | ΔH (kJ/mol $_{CO2}$) |
| MEA | 30 % poids | 40 | 0,50 | 0,41 | 0,47 | 92 |
| N,N,N',N'-(tétraméthyl)-1,6-diamino-2,5-hexanediol (selon l'invention) | 49% poids | 40 | 0.62 | 0.18 | 1.07 | 74 |
| N,N,N',N'-(tétraméthyl)-1,8-diamino-2,7-octanediol (selon l'invention) | 47 % poids | 40 | 0.70 | 0.19 | 1.02 | 73 |

Pour une application captage des fumées en post-combustion où la pression partielle de $CO_2$ dans l'effluent à traiter est de 0,1 bar, cet exemple illustre la plus grande capacité cyclique obtenue grâce à des solutions absorbantes de N,N,N',N'-(tétraméthyl)-1,6-diamino-2,5-hexanediol à 49%poids et de N,N,N',N'-(tétraméthyl)-1,8-diamino-2,7-octanediol à 47 % poids selon l'invention, pour atteindre un taux d'abattage de 90 % en sortie d'absorbeur. Dans cette application où l'énergie associée à la régénération de la solution est critique, on peut remarquer que l'amine selon l'invention permet d'obtenir un bien meilleur compromis que la MEA, en termes de capacité cyclique et d'enthalpie de réaction.

**Revendications**

1. Composé azoté appartenant à la famille des diamines tertiaires répondant à la formule générale (I) suivante :

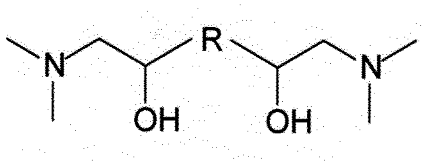

(I)

dans laquelle R est un radical alcanediyl $-(CH_2)n-$ avec n = 2, 3, 4, 5 ou 6.

2. Composé azoté selon la revendication 1, dans lequel n est égal à 2 et R est un radical éthylidène r1, répondant au nom de N,N,N',N'-(tétraméthyl)-1,6-diamino-2,5-hexanediol et à la formule suivante :

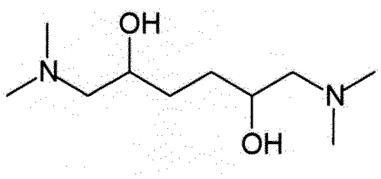

3. Composé azoté selon la revendication 1, dans lequel n est égal à 4 et R est radical butylidène r2, répondant au nom de N,N,N',N'-(tétraméthyl)-1,8-diamino-2,7-octanediol et à la formule suivante :

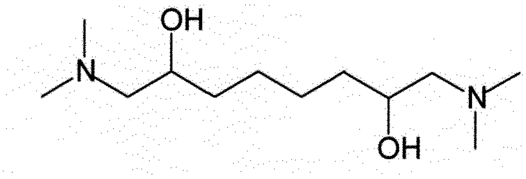

4. Procédé de synthèse d'un composé azoté selon la revendication 1, comprenant les réactions suivantes :

- une première réaction d'époxydation d'un alpha-oméga-diène pour effectuer l'époxydation de chacune des fonctions alcènes de l'alpha-oméga-diène en fonctions oxiranes afin de produire un diépoxyalcane;
- une deuxième réaction d'addition de deux moles de diméthylamine et d'une molécule du diépoxyalcane pour produire le composé azoté selon la revendication 1.

5. Procédé selon la revendication 4, dans lequel :

- la première réaction est une réaction d'époxydation du 1,5-hexadiène pour produire du 1,2,5,6-diépoxyhexane ;
- la deuxième réaction est une réaction d'addition de deux moles de diméthylamine et d'une molécule de 1,2,5,6-diépoxyhexane pour produire du N,N,N',N'-(tétraméthyl)-1,6-diamino-2,5-hexanediol.

6. Procédé selon la revendication 4, dans lequel :

- la première réaction est une réaction d'époxydation du 1,7-octadiène pour produire du 1,2,7,8-diépoxyoctane ;
- la deuxième réaction est une réaction d'addition de deux moles de diméthylamine et d'une molécule de 1,2,7,8-diépoxyoctane pour produire du N,N,N',N'-(tétraméthyl)-1,8-diamino-2,7-octanediol.

7. Procédé d'élimination des composés acides contenus dans un effluent gazeux dans lequel on effectue une étape d'absorption des composés acides par mise en contact de l'effluent gazeux avec une solution absorbante comportant de l'eau et un composé azoté selon l'une des revendications 1 à 3 ou susceptible d'être obtenu par un procédé de synthèse selon l'une des revendications 4 à 6.

8. Procédé selon la revendication 7, dans lequel la solution absorbante comporte entre 5 % et 95 % poids dudit composé azoté, de préférence entre 10 % et 90 % poids dudit composé azoté, et entre 5 % et 95 % poids d'eau, et de préférence entre 10 % et 90% poids d'eau.

9. Procédé selon l'une des revendications 7 et 8, dans lequel la solution absorbante comporte en outre entre 5 % et 95 % poids d'au moins une amine supplémentaire, ladite amine supplémentaire étant soit une amine tertiaire, soit une amine secondaire comprenant deux carbones secondaires en alpha de l'atome d'azote ou au moins un carbone tertiaire en alpha de l'atome d'azote.

10. Procédé selon la revendication 9, dans lequel ladite amine supplémentaire est une amine tertiaire choisie dans le groupe constitué par :

- la N-méthyldiéthanolamine ;
- la triéthanolamine ;
- la diéthylmonoéthanolamine ;
- la diméthylmonoéthanolamine ; et
- l'éthyldiéthanolamine.

11. Procédé selon l'une des revendications 7 à 10, dans lequel la solution absorbante comporte en outre une quantité non nulle et inférieure à 30 % poids d'au moins une amine supplémentaire étant une amine primaire ou une amine secondaire.

12. Procédé selon la revendication 11, dans lequel ladite amine supplémentaire primaire ou secondaire est choisie dans le groupe constitué par :

- la monoéthanolamine ;
- la diéthanolamine ;

- la N-butyléthanolamine ;
- l'aminoéthyléthanolamine ;
- le diglycolamine ;
- la pipérazine ;
- la 1-méthylpipérazine ;
- la 2-méthylpipérazine ;
- l'homopipérazine ;
- la N-(2-hydroxyéthyl)pipérazine ;
- la N-(2-aminoéthyl)pipérazine ;
- la morpholine ;
- la 3-(méthylamino)propylamine ;
- la 1,6-hexanediamine ;
- la N,N,-diméthyl-1,6-hexanediamine ;
- la N,N'-diméthyl-1,6-hexanediamine ;
- la N-méthyl-1,6-hexanediamine ; et
- la N,N',N'-triméthyl-1,6-hexanediamine.

**13.** Procédé selon l'une des revendications 7 à 12 dans lequel la solution absorbante comporte en outre au moins un solvant physique choisi dans le groupe constitué par le méthanol, l'éthanol, le 2-éthoxyéthanol, le triéthylèneglycoldiméthyléther, le tétraéthylèneglycoldiméthyléther, le pentaéthylèneglycoldiméthyléther, l'hexaéthylèneglycoldiméthyléther, l' heptaéthylèneglycoldiméthyléther, l'octaéthylèneglycoldiméthyléther, le butoxyacétate de diéthylèneglycol, le triacétate de glycérol, le sulfolane, la N-méthylpyrrolidone, la N-méthylmorpholin-3-one, le N,N-diméthylformamide, la N-formyl-morpholine, la N,N-diméthyl-imidazolidin-2-one, le N-méthylimidazole, l'éthylèneglycol, le diéthylèneglycol, le triéthylèneglycol, le thiodiglycol, et le tributylphosphate.

**14.** Procédé selon l'une des revendications 7 à 13, dans lequel l'effluent gazeux choisi est parmi le gaz naturel, les gaz de synthèse, les fumées de combustion, les gaz de raffinerie, les gaz acides issus d'une unité aux amines, les gaz issus d'une unité de réduction en queue du procédé Claus, les gaz de fermentation de biomasse, les gaz de cimenterie, les fumées d'incinérateur.

**15.** Procédé selon l'une des revendications 7 à 14 mis en oeuvre pour l'élimination sélective de l'$H_2S$ par rapport au $CO_2$ d'un effluent gazeux comportant de l'$H_2S$ et du $CO_2$, de préférence du gaz naturel.

## Patentansprüche

**1.** Stickstoffverbindung, welche zur Familie der tertiären Diamine gehört, mit der folgenden allgemeinen Formel (I):

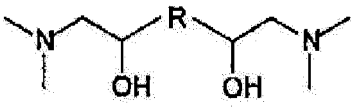

(I)

wobei R ein Rest Alkandiyl-$(CH_2)_n$- ist, wobei n = 2, 3, 4, 5 oder 6.

**2.** Stickstoffverbindung nach Anspruch 1, wobei n gleich 2 ist, und R ein Ethylidenrest r1 mit dem Namen N,N,N',N'-(Tetramethyl)-1,6-diamino-2,5-hexandiol und mit der folgenden Formel ist:

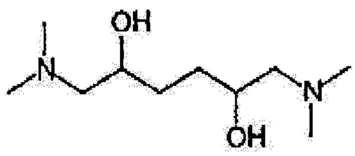

**3.** Stickstoffverbindung nach Anspruch 1, wobei n gleich 4 ist, und R ein Butylidenrest r2 mit dem Namen N,N,N',N'-(Tetramethyl)-1,8-diamino-2,7-octandiol und mit der folgenden Formel ist:

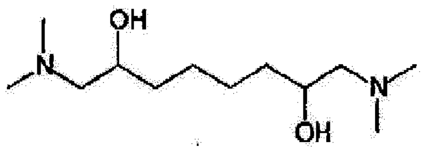

4. Verfahren zur Synthese einer Stickstoffverbindung nach Anspruch 1, umfassend die folgenden Reaktionen:

- eine erste Epoxydationsreaktion eines alpha-omega-Diens, um die Epoxydation jeder der Alkenfunktionen des alpha-omega-Diens in Oxiranfunktionen zu bewirken, um ein Diepoxyalkan zu erzeugen;
- eine zweite Additionsreaktion von zwei Mol Dimethylamin und einem Molekül des Diepoxyalkans, um die Stickstoffverbindung nach Anspruch 1 zu erzeugen.

5. Verfahren nach Anspruch 4, wobei:

- die erste Reaktion eine Epoxydationsreaktion von 1,5-Hexandien ist, um 1,2,5,6-Diepoxyhexan zu erzeugen;
- die zweite Reaktion eine Additionsreaktion von zwei Mol Dimethylamin und einem Molekül von 1,2,5,6-Diepoxyhexan ist, um N,N,N',N'-(Tetramethyl)-1,6-diamino-2,5-hexandiol zu erzeugen.

6. Verfahren nach Anspruch 4, wobei:

- die erste Reaktion eine Epoxydationsreaktion von 1,7-Octadien ist, um 1,2,7,8-Diepoxyoctan zu erzeugen;
- die zweite Reaktion eine Additionsreaktion von zwei Mol Dimethylamin und einem Molekül von 1,2,7,8-Diepoxyoctan ist, um N,N,N',N'-(Tetramethyl)-1,8-diamino-2,7-octandiol zu erzeugen.

7. Verfahren zur Eliminierung von sauren Verbindungen, die in einem Gasstrom enthalten sind, wobei ein Schritt der Absorption von sauren Verbindungen durchgeführt wird, indem der Gasstrom mit einer absorbierenden Lösung in Kontakt gebracht wird, die Wasser und eine Stickstoffverbindung nach einem der Ansprüche 1 bis 3, oder die durch ein Syntheseverfahren nach einem der Ansprüche 4 bis 6 erhalten werden kann, umfasst.

8. Verfahren nach Anspruch 7, wobei die absorbierende Lösung zwischen 5 Gew.-% und 95 Gew.-% der Stickstoffverbindung, vorzugsweise zwischen 10 Gew.-% und 90 Gew.-% der Stickstoffverbindung, und zwischen 5 Gew.-% und 95 Gew.-% Wasser, und vorzugsweise zwischen 10 Gew.-% und 90 Gew.-% Wasser, umfasst.

9. Verfahren nach einem der Ansprüche 7 und 8, wobei die absorbierende Lösung außerdem zwischen 5 Gew.-% und 95 Gew.-% mindestens eines ergänzenden Amins umfasst, wobei das ergänzende Amin entweder ein tertiäres Amin oder ein sekundären Amin mit zwei sekundären Kohlenstoffen in Stellung alpha des Stickstoffatoms oder mindestens einem tertiären Kohlenstoff in Stellung alpha des Stickstoffatoms ist.

10. Verfahren nach Anspruch 9, wobei das ergänzende Amin ein tertiäres Amin ist, ausgewählt aus der Gruppe bestehend aus:

- N-Methyldiethanolamin;
- Triethanolamin;
- Diethylmonoethanolamin;
- Dimethylmonoethanolamin; und
- Ethyldiethanolamin.

11. Verfahren nach einem der Ansprüche 7 bis 10, wobei die absorbierende Lösung außerdem eine Menge von nicht Null und weniger als 30 Gew.-% mindestens eines ergänzenden Amins umfasst, das ein primäres Amin oder ein sekundäres Amin ist.

12. Verfahren nach Anspruch 11, wobei das ergänzende primäre oder sekundäre Amin ausgewählt wird aus der Gruppe bestehend aus:

- Monoethanolamin;
- Diethanolamin;
- N-Butylethanolamin;

- Aminoethylethanolamin;
- Diglykolamin;
- Piperazin;
- 1-Methylpiperazin;
- 2-Methylpiperazin;
- Homopiperazin;
- N-(2-Hydroxyethyl)-piperazin;
- N-(2-Aminoethyl)-piperazin;
- Morpholin;
- 3-(Methylamino)-propylamin;
- 1,6-Hexandiamin;
- N,N-Dimethyl-1,6-hexandiamin;
- N,N'-Dimethyl-1,6-hexandiamin;
- N-Methyl-1,6-hexandiamin; und
- N,N',N'-Trimethyl-1,6-hexandiamin.

**13.** Verfahren nach einem der Ansprüche 7 bis 12, wobei die absorbierende Lösung außerdem mindestens ein physikalisches Lösungsmittel umfasst, ausgewählt aus der Gruppe bestehend aus Methanol, Ethanol, 2-Ethoxyethanol, Triethylenglykoldimethylether, Tetraethylenglykoldimethylether, Pentaethylenglykoldimethylether, Hexaethylenglykoldimethylether, Heptaethylenglykoldimethylether, Octaethylenglykoldimethylether, Diethylenglykolbutoxyacetat, Glycerintriacetat, Sulfolan, N-Methylpyrrolidon, N-Methylmorpholin-3-on, N,N-Dimethylformamid, N-Formylmorpholin, N,N-Dimethylimidazolidin-2-on, N-Methylimidazol, Ethylenglykol, Diethylenglykol, Tetraethylenglykol, Thiodiglykol und Tributylphosphat.

**14.** Verfahren nach einem der Ansprüche 7 bis 13, wobei der Gasstrom ausgewählt wird aus Erdgas, Synthesegasen, Verbrennungsgasen, Raffineriegasen, sauren Gasen, die aus einer Einheit für Amine stammen, Gase, die aus einer Stufenreduktionseinheit des Claus-Prozesses stammen, Biomassefermentationsgasen, Zementfabriksgasen, Verbrennungsofenabgasen.

**15.** Verfahren nach einem der Ansprüche 7 bis 14, welches zur selektiven Eliminierung von $H_2S$ im Verhältnis zu $CO_2$ aus einem Gasstrom, der $H_2S$ und $CO_2$ umfasst, vorzugsweise Erdgas, durchgeführt wird.

## Claims

**1.** A nitrogen compound belonging to the family of tertiary diamines meeting general formula (I) as follows:

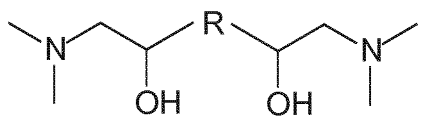

(I)

wherein R is an alkanediyl radical $-(CH_2)n-$ with n = 2, 3, 4, 5 or 6.

**2.** A nitrogen compound as claimed in claim 1, wherein n is equal to 2 and R is an ethylidene radical r1, named N,N,N',N'-(tetramethyl)-1,6-diamino-2,5-hexanediol and meeting the formula as follows:

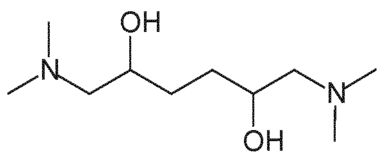

**3.** A nitrogen compound as claimed in claim 1, wherein n is equal to 4 and R is a butylidene radical r2, named N,N,N',N'-(tetramethyl)-1,8-diamino-2,7-octanediol and meeting the formula as follows:

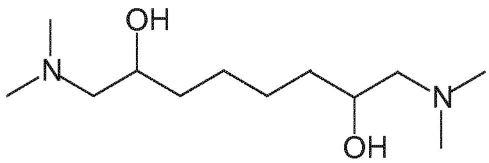

**4.** A synthesis method of a nitrogen compound as claimed in claim 1, comprising the following reactions:

- a first reaction of epoxidation of an alpha-omega-diene to achieve epoxidation of each one of the alkene functions of the alpha-omega-diene to oxirane functions so as to produce a diepoxyalkane,
- a second reaction of addition of two moles of dimethylamine and one molecule of the diepoxyalkane so as to produce the nitrogen compound as claimed in claim 1.

**5.** A method as claimed in claim 4, wherein:

- the first reaction is an epoxidation reaction of 1,5-hexadiene to produce 1,2,5,6-diepoxyhexane,
- the second reaction is an addition reaction of two moles of dimethylamine and one molecule of 1,2,5,6-diepoxyhexane to produce N,N,N',N'-(tetramethyl)-1,6-diamino-2,5-hexanediol.

**6.** A method as claimed in claim 4, wherein:

- the first reaction is an epoxidation reaction of 1,7-octadiene to produce 1,2,7,8-diepoxyoctane,
- the second reaction is an addition reaction of two moles of dimethylamine and one molecule of 1,2,7,8-diepoxyoctane to produce N,N,N',N'-(tetramethyl)-1,8-diamino-2,7-octanediol.

**7.** A method of removing acid compounds contained in a gaseous effluent wherein an acid compound absorption stage is carried out by contacting the gaseous effluent with an absorbent solution comprising water and a nitrogen compound as claimed in any one of claims 1 to 3 or likely to be obtained by a synthesis method as claimed in any one of claims 4 to 6.

**8.** A method as claimed in claim 7, wherein the absorbent solution comprises between 5 wt.% and 95 wt.% of said nitrogen compound, preferably between 10 wt.% and 90 wt.% of said nitrogen compound, and between 5 wt.% and 95 wt.% of water, preferably between 10 wt.% and 90 wt.% of water.

**9.** A method as claimed in any one of claims 7 and 8, wherein the absorbent solution additionally comprises between 5 wt.% and 95 wt.% of at least one additional amine, said additional amine being either a tertiary amine or a secondary amine having two secondary carbons at nitrogen alpha position or at least one tertiary carbon at nitrogen alpha position.

**10.** A method as claimed in claim 9, wherein said additional amine is a tertiary amine selected from among the group made up of:

- N-methyldiethanolamine,
- triethanolamine,
- diethylmonoethanolamine,
- dimethylmonoethanolamine, and
- ethyldiethanolamine.

**11.** A method as claimed in any one of claims 7 to 10, wherein the absorbent solution also comprises a non-zero amount less than 30 wt.% of at least one additional amine such as a primary amine or a secondary amine.

**12.** A method as claimed in claim 11, wherein said additional primary or secondary amine is selected from among the group made up of:

- monoethanolamine,
- diethanolamine,
- N-butylethanolamine,

- aminoethylethanolamine,
- diglycolamine,
- piperazine,
- 1-methylpiperazine,
- 2-methylpiperazine,
- homopiperazine,
- N-(2-hydroxyethyl)piperazine,
- N-(2-aminoethyl)piperazine,
- morpholine,
- 3-(methylamino)propylamine,
- 1,6-hexanediamine,
- N,N-dimethyl-1,6-hexanediamine,
- N,N'-dimethyl-1,6-hexanediamine,
- N-methyl-1,6-hexane-diamine, and
- N,N',N'-trimethyl-1,6-hexanediamine.

**13.** A method as claimed in any one of claims 7 to 12, wherein the absorbent solution furthermore comprises at least one physical solvent selected from the group made up of methanol, ethanol, 2-ethoxyethanol, triethylene glycoldimethylether, tetra-ethylene glycoldimethylether, pentaethylene glycoldimethylether, hexaethylene glycoldimethylether, heptaethylene glycol-dimethylether, octaethylene glycol-dimethylether, diethylene glycol butoxyacetate, glycerol triacetate, sulfolane, N-methylpyrrolidone, N-methylmorpholin-3-one, N,N-dimethylformamide, N-formyl-morpholine, N,N-dimethyl-imidazolidin-2-one, N-methylimidazole, ethylene glycol, diethylene glycol, triethylene glycol, thiodiglycol and tributyl phosphate.

**14.** A method as claimed in any one of claims 7 to 13, wherein the gaseous effluent is selected from among natural gas, syngases, combustion fumes, refinery gas, acid gas from an amine plant, Claus tail gas, biomass fermentation gas, cement plant gas and incinerator fumes.

**15.** A method as claimed in any one of claims 7 to 14, implemented for selectively removing the $H_2S$ over the $CO_2$ from a gaseous effluent comprising $H_2S$ and $CO_2$, preferably natural gas.

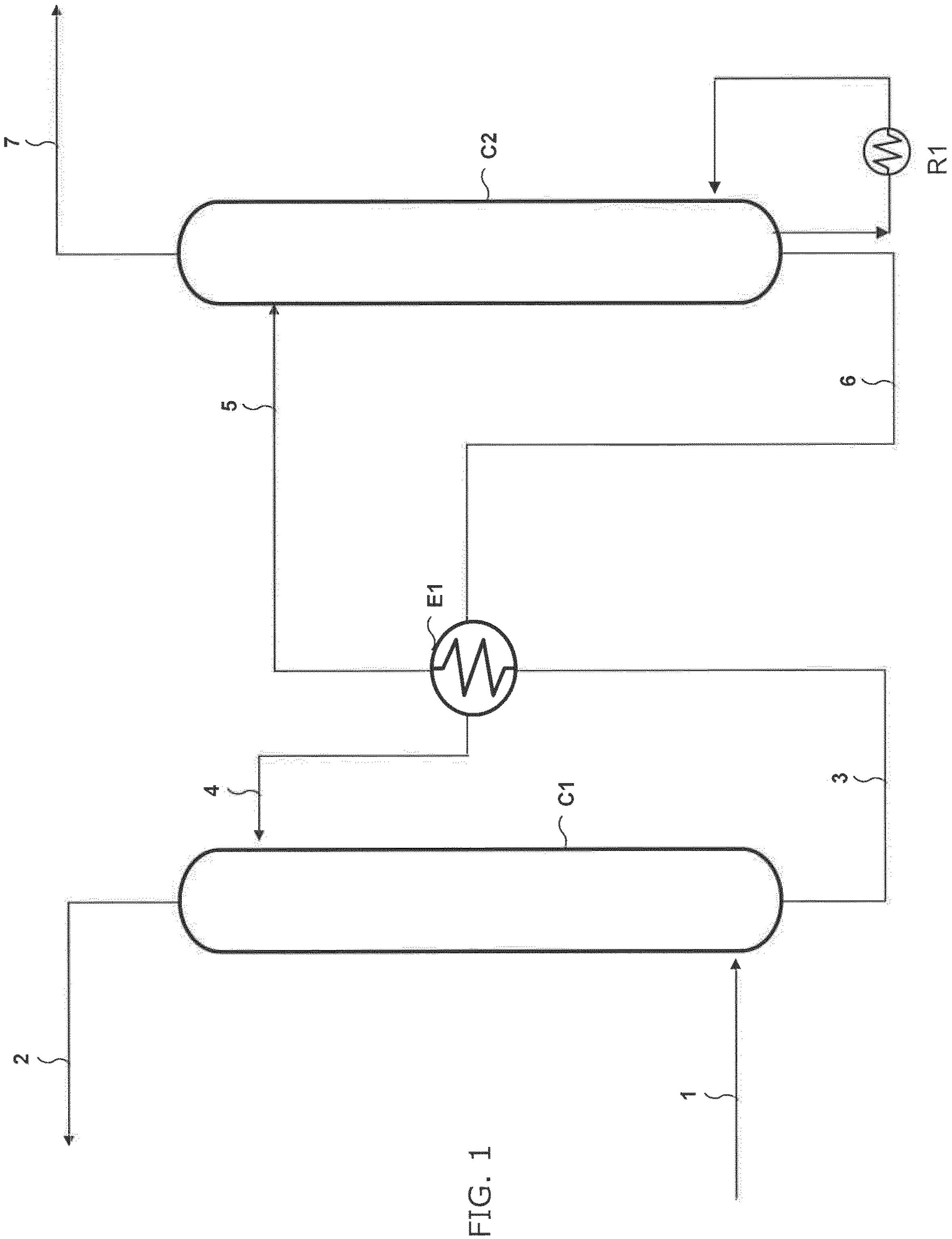


FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6852144 B
- US 4405582 A
- US 4405583 A
- FR 2934172
- WO 2013060944 A
- US 2013243677 A
- US 2010105551 A
- US 5277885 A